(19) **Europäisches Patentamt / European Patent Office / Office européen des brevets**

(11) **EP 4 769 083 A1**

(12) **DEMANDE DE BREVET EUROPEEN**

(43) Date de publication:
**01.07.2026 Bulletin 2026/27**

(21) Numéro de dépôt: **25227311.5**

(22) Date de dépôt: **26.12.2025**

(51) Classification Internationale des Brevets (IPC):
**G06F 3/01** (2006.01)     **A61B 5/00** (2006.01)
**A61B 5/372** (2021.01)

(52) Classification Coopérative des Brevets (CPC):
**G06F 3/015; A61B 5/372; A61B 5/7267;**
A61B 5/291; A61B 2562/046

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH LA MA MD TN**

(30) Priorité: **27.12.2024 FR 2415312**

(71) Demandeur: **Commissariat à l'Energie Atomique et aux Energies Alternatives**
**75015 Paris (FR)**

(72) Inventeurs:
• **LAFAYE DE MICHEAUX, Hugo**
  **38054 GRENOBLE CEDEX 09 (FR)**
• **MARTEL, Félix**
  **38054 GRENOBLE CEDEX 09 (FR)**
• **AKSENOVA, Tétiana**
  **38054 GRENOBLE CEDEX 09 (FR)**

(74) Mandataire: **INNOV-GROUP**
**209 Avenue Berthelot**
**69007 Lyon (FR)**

(54) **PROCÉDÉ D'APPRENTISSAGE EN LIGNE D'UNE INTERFACE NEURONALE**

(57) Procédé d'apprentissage d'une interface neuronale directe, l'interface neuronale directe étant reliée à des capteurs ($2_1...2_{I1}$) préalablement disposés autour du cerveau d'un utilisateur, l'interface étant configurée pour commander un actionneur (6) en fonction de signaux électrophysiologiques détectés par chaque capteur, en appliquant un modèle prédictif à un tenseur d'observation formé par les signaux détectés durant une époque temporelle. L'interface neuronale comporte un modèle de décodage d'un état de satisfaction permettant d'estimer une satisfaction de l'utilisateur à partir de signaux électrophysiologiques détectés à chaque époque temporelle. Le procédé est tel que le décodeur est formé en fonction d'un poids respectivement assigné à chaque époque temporelle.

Fig. 2A

EP 4 769 083 A1

**Fig. 2B**

**Fig. 2C**

**Description**

**DOMAINE TECHNIQUE**

Le domaine technique de l'invention concerne les interfaces neuronales directes, usuellement désignées par le terme « BCI » (Brain Computer Interface), destinées à commander, à partir de signaux neurophysiologiques, un actionneur.

**ART ANTERIEUR**

Le domaine des interfaces neuronales directes est en plein développement et apparaît comme une solution séduisante pour permettre à des utilisateurs en situation de handicap de commander des actionneurs par la pensée. Il s'agit de détecter et d'enregistrer des signaux électrophysiologiques émis par le cortex. Ces derniers sont traités par des algorithmes permettant de former un signal de commande, pour commander des actionneurs. Le signal de commande peut permettre le pilotage d'un exosquelette, d'un ordinateur ou d'un robot, pour fournir une assistance à l'utilisateur. Les algorithmes mis en œuvre permettent une traduction d'une instruction donnée par l'utilisateur, cette instruction étant captée, par des électrodes, sous la forme des signaux dits électrophysiologiques, car représentatifs de l'activité électrique des neurones. Cette activité électrique est peut être au niveau du cortex, au moyen d'électrodes corticales disposées dans la boîte crânienne. Elle peut également être mesurée par des électrodes d'électroencéphalographie, moins intrusives car disposées sur le scalp, mais également moins performantes, notamment au niveau de la résolution spatiale. Une autre solution est d'enregistrer les signaux électrophysiologiques par magnétoencéphalographie, ce qui nécessite une installation dédiée.

Les algorithmes mis en œuvre sont généralement basés sur un modèle prédictif. Le modèle prédictif utilise des données d'entrée, obtenues par un prétraitement des signaux électrophysiologiques enregistrés, pour établir un signal de commande, à destination de l'actionneur ou des actionneurs. Le signal de commande doit correspondre à une intention exprimée par l'utilisateur, dont on enregistre les signaux électrophysiologiques. L'intention exprimée par l'utilisateur se manifeste sous la forme des signaux électrophysiologiques, ces derniers étant enregistrés et transmis vers l'interface neuronale directe, formant des données d'observations. Les signaux électrophysiologiques sont traités pour obtenir des données d'observations, formant des données d'entrée du modèle, ce dernier générant un signal de commande correspondant à l'intention exprimée par l'utilisateur. Le signal de commande permet une commande de l'actionneur ou d'un membre de l'utilisateur.

Les données d'observation sont généralement multidimensionnelles, et comprennent :

- une composante spatiale, représentative de l'origine spatiale du signal électrophysiologique ;
- une composante fréquentielle, représentative de l'intensité du signal électrophysiologique dans différentes bandes de fréquence ;
- une composante temporelle qui correspond à un échantillon temporel du signal électrophysiologique dans lequel l'analyse fréquentielle est effectuée.

Chaque donnée d'observation est associée à une époque, c'est-à-dire à un échantillon temporel de durée prédéterminée, par exemple de l'ordre de 1 seconde après que l'utilisateur ait eu l'intention d'effectuer la tâche. Le terme époque correspond à l'équivalent du terme anglosaxon « epoch ». A chaque époque, on forme un tenseur d'observation, rassemblant les données d'observation. A partir du tenseur d'observation, on alimente un modèle prédictif. Le modèle prédictif, appliqué au tenseur d'observation, permet d'estimer un signal de commande, permettant la commande des actionneurs. Le signal de commande est généralement exprimé par un vecteur de commande.

Le modèle prédictif est établi au cours d'une phase d'apprentissage, au cours de laquelle l'utilisateur effectue des tâches prédéfinies, pour lesquelles la sortie du modèle prédictif est connue. La sortie du modèle prédictif correspond à un vecteur de commande connu, à partir duquel on détermine la tâche à exécuter la plus probable. L'objectif de l'apprentissage est de déterminer des composantes des signaux électrophysiologiques enregistrés spécifiques de chaque tâche. Il peut notamment s'agir de déterminer des corrélations entre des composantes des signaux électrophysiologiques et la sortie du modèle.

L'établissement de modèles prédictifs a amplement été décrit. Par exemple, le brevet US9480583 décrit l'application d'une méthode de régression linéaire des moindres carrés partiels multivariée permettant d'établir un modèle prédictif. Une telle méthode est connue sous l'acronyme anglosaxon "NPLS" ou par le terme "N-way Partial Least Squares". L'application d'une telle méthode a également été décrite dans la publication Eliseyev A, Aksenova T (2013) "Recursive N-way Partial Least Squares for Brain Computer Interface" PIOS ONE july 2013, Volume 8 Issue 7. Une telle méthode est également décrite dans le document Yelisyeyev A « Brain-Computer Interface with cortical electrical activity recording" Human health and pathology ». Université de Grenoble, 2011.

Cependant, le recours à une méthode de type NPLS nécessite de traiter un grand nombre de données d'apprentissage, par exemple plusieurs centaines ou plusieurs milliers pour une sortie du modèle correspondant à une tâche spécifique. Cela suppose un stockage en mémoire d'une quantité d'information élevée, ce qui n'est pas approprié pour effectuer un apprentissage en ligne, c'est-à-dire en temps réel, ou en quasi temps réel. Par quasi temps réel, on entend un apprentissage effectué par séquences successives, chaque séquence durant quelques secondes ou quelques minutes.

Afin de diminuer la quantité d'information à mémoriser, un procédé d'apprentissage récursif mettant en œuvre une méthode de type REW-NPLS a été développée, REW signifiant « Recursive Exponentially Weighted », ou pondération récursive exponentielle. La formation d'un modèle prédictif, par REW-NPLS, appliqué à une interface BCI, est décrit dans EP3985530. L'approche récursive est justifiée par le fait que les signaux neuronaux ne sont pas stationnaires, ce qui impose une mise à jour régulière du modèle prédictif.

Les procédés d'apprentissage précédemment cités sont mis en œuvre de façon supervisée : l'utilisateur suit des consignes lui demandant d'exécuter une tâche choisie parmi une liste de tâches prédéfinies, et correspondant à un vecteur de commande prédéterminé.

Le brevet EP4024170 ainsi que la publication Rouanne, « Unsupervised adaptation of an ECoG based brain-computer interface using neural correlates of task performance », Nature scientific reports (2012) 12 :21316, décrit un apprentissage, comportant une prise en compte d'un état de satisfaction de l'utilisateur par rapport au signal de commande généré par le modèle prédictif. Cela permet une mise à jour d'un modèle prédictif en annulant ou de pondérant un signal de commande en fonction d'un état de satisfaction de l'utilisateur.

Les inventeurs proposent une amélioration de la méthode décrite dans EP3985530, de façon à améliorer la performance d'apprentissage du modèle prédictif et/ou la performance de décodage de l'état de satisfaction de l'utilisateur. L'objectif est d'améliorer l'apprentissage non supervisé du modèle prédictif mis en œuvre par l'interface.

**EXPOSE DE L'INVENTION**

Un premier objet de l'invention est un procédé d'apprentissage d'une interface neuronale directe, l'interface neuronale directe comportant des capteurs préalablement disposés autour du cerveau d'un utilisateur, et configurés pour détecter des signaux électrophysiologiques, représentatifs d'une activité neuronale de l'utilisateur, l'interface étant configurée pour :

- commander un actionneur, en mettant en œuvre un modèle prédictif de commande, le modèle prédictif de commande étant configuré pour générer un signal de commande de l'actionneur à partir de signaux électrophysiologiques détectés ;
- estimer un état de satisfaction de l'utilisateur en mettant en œuvre un modèle de décodage, le modèle de décodage étant configuré pour estimer l'état de satisfaction de l'utilisateur à partir de signaux électrophysiologiques détectés ;

le procédé comportant les étapes suivantes :

a) choix d'une tâche à effectuer, choisie parmi une liste de tâches prédéterminée;

b) instruction, à l'utilisateur, d'imaginer une exécution de la tâche choisie lors de l'étape a) et suite à l'instruction, à l'aide de l'unité de traitement:

- acquisition des signaux électrophysiologiques moteurs, et formation d'un tenseur d'observation à partir de caractéristiques des signaux électrophysiologiques;
- application du modèle prédictif de commande au tenseur d'observation, pour générer un signal de commande, pour piloter l'actionneur, le signal de commande étant représentatif d'une tâche prédite ;

c) réitération des étapes a) et b) durant plusieurs époques temporelles, à chaque époque étant assignée une tâche choisie lors de l'étape a) et une tâche prédite durant l'étape b) ;

d) sélection d'époques auxquelles la tâche prédite lors de l'étape b) est identiques aux tâches prédites lors de l'étape b) d'un nombre minimal d'époques précédentes successives, les époques ainsi sélectionnées formant une séquence.

Le procédé peut comporter :

- e) pour chaque époque sélectionnée dans l'étape d), assignation d'un label représentant une comparaison de la tâche prédite lors de l'étape b), avec la tâche choisie lors de l'étape a), le label étant choisi parmi plusieurs valeurs possibles;
- f) formation d'un vecteur de labels à partir des labels assignés lors de l'étape e) ;

- g) formation d'un tenseur d'apprentissage à partir des tenseurs d'observation formés pour chaque époque sélectionnée dans l'étape d) ;
- h) mise à jour du modèle de décodage par régression entre le vecteur de labels et le tenseur d'apprentissage ;

le procédé étant caractérisé en ce que l'étape h) comporte :

- définition d'un critère de pondération pour chaque époque sélectionnée ;
- assignation d'un poids à chaque époque sélectionnée, le poids étant défini en fonction du critère de pondération, en fonction duquel à deux époques différentes, de la séquence, pour lesquelles le critère de pondération est différent, sont assignés deux poids différents ;

et en ce que la formation du modèle de décodage est effectuée en fonction du poids respectivement assigné à chaque époque sélectionnée.

Selon une possibilité, le poids assigné à une époque dépend de la tâche sélectionnée durant ladite époque.

Selon une possibilité, l'étape h) comporte :

- hi) subdivision de chaque tâche, respectivement associée à chaque époque sélectionnée, en plusieurs sous-classes, correspondant respectivement à chaque valeur possible du label suite à l'étape e), de façon que la sous-classe attribuée à chaque époque corresponde à la valeur prise par le label pour la tâche choisie lors de l'étape a) de ladite époque ;
- hii) assignation d'un poids à chaque sous-classe, le poids étant défini en fonction du critère de pondération;
- hiii) assignation du poids à chaque époque , le poids de chaque époque correspondant au poids assigné, lors de la sous-étape hii), à la sous-classe attribuée à ladite époque lors de la sous-étape hi).

Selon une possibilité:

- les étapes a) à d) sont mises en œuvre durant plusieurs séquences successives ;
- les étapes e) à h) sont est mises en œuvre pour chaque séquence ;
- dans la sous-étape hii), le critère de pondération est une fréquence d'occurrence de chaque sous-classe, le poids de chaque époque est d'autant plus élevé que le nombre d'occurrences de la sous-classe, suite aux séquences successives effectuées, est faible.

Selon une possibilité, après chaque nouvelle séquence, le procédé comporte une mise à jour d'un nombre total d'occurrences pondéré pour chaque sous-classe, la mise à jour comportant, pour chaque sous-classe :

- détermination d'un nombre d'occurrences dans la nouvelle séquence ;
- pondération du nombre d'occurrences, au cours de la nouvelle séquence, par le poids respectivement assigné à sous-classe dans la nouvelle séquence ;
- sommation du nombre d'occurrences pondéré de la sous-classe, pour la nouvelle séquence, au nombre total pondéré pour chaque sous-classe résultant de la séquence précédente, ce dernier étant multiplié par un facteur d'oubli.

Selon une possibilité, le modèle de décodage est défini par régression multivariée, comportant un calcul d'un tenseur de covariance croisée entre le tenseur d'apprentissage et le vecteur de labels. Le tenseur de covariance croisé de chaque séquence est établi à partir d'un produit :

- du tenseur d'apprentissage ;
- du vecteur de labels;
- des poids assignés à chaque époque.

Selon une possibilité :

- les étapes a) à d) sont répétées de façon à former plusieurs séquences successives, à chaque séquence étant un rang chronologique;
- les étapes e) à h) sont est mises en œuvre pour chaque séquence ;
- lors de l'étape h), le modèle de décodage est établi à partir de deux séquences consécutives, à partir d'une somme du tenseur de covariance croisée établi pour la séquence de rang supérieur et du tenseur de covariance croisée établi pour la séquence de rang inférieur multiplié par un facteur d'oubli.

Selon une possibilité :

- le tenseur d'apprentissage prend la forme d'une matrice, dont une dimension est le nombre d'époques par séquence ;
- dans chaque étape h), relative à chaque séquence, les poids forment une matrice diagonale , chaque terme de la matrice diagonale correspondant au poids assigné à l'époque respectivement exécutée lors de ladite séquence.

Selon une possibilité, pouvant être mise en œuvre indépendamment des étapes e), f), g) et la régression de l'étape h), le procédé comporte également :

- j-i) formation d'un tenseur d'observation auxiliaire à partir des signaux électrophysiologiques détectés lors de chaque époque sélectionnée dans l'étape d) ;
- j-ii) application d'un modèle de décodage au tenseur d'observation auxiliaire de façon à estimer un label pour chaque époque ;
- j-iii) pondération de la valeur du label estimé pour chaque époque, par le poids assigné à chaque époque lors de l'étape h) ;
- j-iv) formation d'un histogramme des valeurs pondérées de chaque label estimé ;
- j-v) calcul d'un fractile inférieur et d'un fractile supérieur à partir de l'histogramme ;
- j-vi) association d'un seuil inférieur et d'un seuil supérieur respectivement à partir du fractile inférieur et du fractile supérieur, de façon qu'un label estimé dont la valeur est inférieure ou supérieure respectivement au seuil inférieur ou au seuil supérieur est considéré comme un label ayant une valeur d'erreur ou une valeur correcte ; les étapes j -i) à j-vi) étant mise en œuvre par l'unité de traitement.

Pour chaque époque sélectionnée lors de l'étape d), le tenseur d'observation et le tenseur d'observation auxiliaire peuvent être identiques.

Un deuxième objet de l'invention est un procédé d'apprentissage d'une interface neuronale directe, l'interface neuronale directe comportant des capteurs préalablement disposés autour du cerveau d'un utilisateur, et configurés pour détecter des signaux électrophysiologiques, représentatifs d'une activité neuronale de l'utilisateur, l'interface étant configurée pour :

- commander un actionneur, en mettant en œuvre un modèle prédictif de commande, le modèle prédictif de commande étant configuré pour générer un signal de commande de l'actionneur à partir de signaux électrophysiologiques détectés ;
- estimer un état de satisfaction de l'utilisateur en mettant en œuvre un modèle de décodage, le modèle de décodage étant configuré pour estimer l'état de satisfaction de l'utilisateur à partir de signaux électrophysiologiques détectés, le modèle de décodage pouvant être établi à partir d'un procédé selon le premier objet de l'invention ;

le procédé comportant :

- i) choix, par l'utilisateur, d'une tache mentale à effectuer, choisie parmi une liste de tâches prédéterminée;
- ii) exécution, par l'utilisateur, de la tâche choisie lors de l'étape i) et, au cours de l'exécution, acquisition des signaux électrophysiologiques, issus des différents capteurs ;
- iii) durant l'exécution de la tâche choisie, mise en œuvre du modèle prédictif de commande, de façon à générer un signal de commande ;
- v) suite à la génération du signal de commande, estimation d'un label, correspondant à un état de satisfaction de l'utilisateur ;
- vi) réitération des étapes i) à v) durant un nombre prédéterminé d'époques temporelles, lesdites époques formant une séquence;
- vii) sélection d'époques en fonction du niveau de satisfaction de l'utilisateur estimé lors de chaque étape v) ;
- viii) formation d'un tenseur d'apprentissage à partir des signaux électrophysiologiques détectés au cours des époques sélectionnées dans l'étape vii) et d'un tenseur de commande à partir des signaux de commande générés au cours des époques sélectionnées lors de l'étape vii) ;
- ix) mise à jour du modèle prédictif de commande en fonction du tenseur d'apprentissage et du tenseur de commande résultant de vii), pour la séquence;

les étapes iii) à ix) étant mises en œuvre par l'unité de traitement à partir de signaux électrophysiologiques détectés ; le procédé étant caractérisé en ce que l'étape ix) comporte :

- définition d'un critère de pondération pour chaque époque sélectionnée dans l'étape vii) ;

- assignation d'un poids à chaque époque, le poids étant défini en fonction du critère de pondération pour ladite époque, en fonction duquel à deux époques différentes, pour lesquelles le critère de pondération est différent, sont assignés deux poids différents ;

le procédé étant tel que la formation du modèle prédictif de commande est effectuée en fonction du poids respectivement assigné à chaque époque.

Le poids assigné à une époque peut dépendre de la tâche (k) choisie durant ladite époque. Selon une possibilité :

- les étapes i) à ix) sont mises en œuvre durant plusieurs séquences successives ;
- le critère de pondération est une fréquence d'occurrence de chaque tâche, le poids de chaque époque est d'autant plus élevé que le nombre d'occurrences de la tâche, suite aux séquences successives effectuées, est faible.

Selon une possibilité, après chaque nouvelle séquence, le procédé comporte une mise à jour d'un nombre total d'occurrences pondéré pour chaque tâche. La mise à jour comporte, pour chaque tâche :

- détermination d'un nombre d'occurrences dans la nouvelle séquence ;
- pondération du nombre d'occurrences, au cours de la nouvelle séquence, par le poids respectivement assigné à la tâche dans la nouvelle séquence ;
- sommation du nombre d'occurrences pondéré de la tâche, pour la nouvelle séquence, au nombre total pondéré pour chaque tâche résultant de la séquence de rang inférieur, ce dernier étant multiplié par un facteur d'oubli.

Selon une possibilité, le critère de pondération est une performance d'apprentissage. Le procédé comporte :

- détermination d'un indicateur de performance d'apprentissage de chaque tâche suite à chaque époque ;
- détermination du poids de chaque tâche en fonction du critère de performance d'apprentissage de la tâche.

Selon une possibilité, le critère de pondération est une qualité des signaux collectés à chaque séquence. Le procédé comporte :

- détermination d'un critère de qualité des signaux collectés à chaque séquence ;
- détermination du poids de chaque tâche en fonction du critère de qualité des signaux.

Selon une possibilité, le modèle prédictif est mis en œuvre par régression multivariée, comportant un calcul d'un tenseur de covariance croisée entre le tenseur d'apprentissage et le tenseur de commande. Le tenseur de covariance croisé de chaque séquence est établi à partir d'un produit :

- du tenseur d'apprentissage;
- du tenseur de commande;
- des poids assignés à chaque époque.

Selon une possibilité, lors de l'étape vii), les époques pour lesquelles le niveau de satisfaction de l'utilisateur sont comprises entre un seuil inférieur et un seuil supérieur sont rejetées.

Un troisième objet de l'invention est une interface neuronale directe, l'interface neuronale directe comportant des capteurs préalablement disposés autour du cerveau d'un utilisateur, et configurés pour détecter des signaux électrophysiologiques, représentatifs d'une activité neuronale de l'utilisateur, l'interface étant configurée pour :

- commander un actionneur, en mettant en œuvre un modèle prédictif de commande, le modèle prédictif étant configuré pour générer un signal de commande de l'actionneur à partir de signaux électrophysiologiques détectés ;
- estimer un état de satisfaction de l'utilisateur en mettant en œuvre un modèle de décodage, le modèle de décodage étant configuré pour estimer l'état de satisfaction de l'utilisateur à partir de signaux électrophysiologiques détectés.

L'interface comporte une unité de traitement, configurée pour acquérir des signaux électrophysiologiques lors de chaque étape b) d'un procédé selon le premier objet de l'invention, et pour mettre en œuvre les étapes c) à h) dudit procédé.

Un quatrième objet de l'invention est interface neuronale directe, l'interface neuronale directe comportant des capteurs préalablement disposés autour du cerveau d'un utilisateur, et configurés pour détecter des signaux électrophysiologiques, représentatifs d'une activité neuronale de l'utilisateur, l'interface étant configurée pour :

- commander un actionneur, en mettant en œuvre un modèle prédictif, le modèle prédictif étant configuré pour générer un signal de commande de l'actionneur à partir de signaux électrophysiologiques détectés ;
- estimer un état de satisfaction de l'utilisateur en mettant en œuvre un modèle de décodage, le modèle de décodage étant configuré pour estimer l'état de satisfaction de l'utilisateur à partir de signaux électrophysiologiques détectés.

L'interface comporte une unité de traitement, configurée pour acquérir des signaux électrophysiologiques lors de chaque étape ii) d'un procédé selon le deuxième objet de l'invention, et pour mettre en œuvre les étapes iii) à ix) dudit procédé.

L'invention sera mieux comprise à la lecture de l'exposé des exemples de réalisation présentés, dans la suite de la description, en lien avec les figures listées ci-dessous.

## FIGURES

La figure 1 schématise une interface neuronale reliée à un utilisateur, et connectée à un processeur apte à mettre en œuvre un procédé selon l'invention.

Les figures 2A, 2B et 2C représentent les principales étapes de blocs permettant une mise en œuvre de l'invention.

La figure 3 représente un histogramme pondéré de grandeurs quantifiant l'état de satisfaction d'un utilisateur suite à la réalisation de plusieurs tâches mentales.

Les figures 4A et 4B montrent une chronologie de chaque session d'apprentissage.

Les figures 5A à 5F montrent une comparaison de performances d'apprentissage en mettant en œuvre un apprentissage supervisé et en mettant en œuvre l'invention pour différents blocs de traitement.

Les figures 6A à 6F montrent une comparaison de performances d'apprentissage en mettant en œuvre un apprentissage supervisé et en mettant en œuvre l'invention entre deux séances.

## EXPOSE DE MODES DE REALISATION PARTICULIER

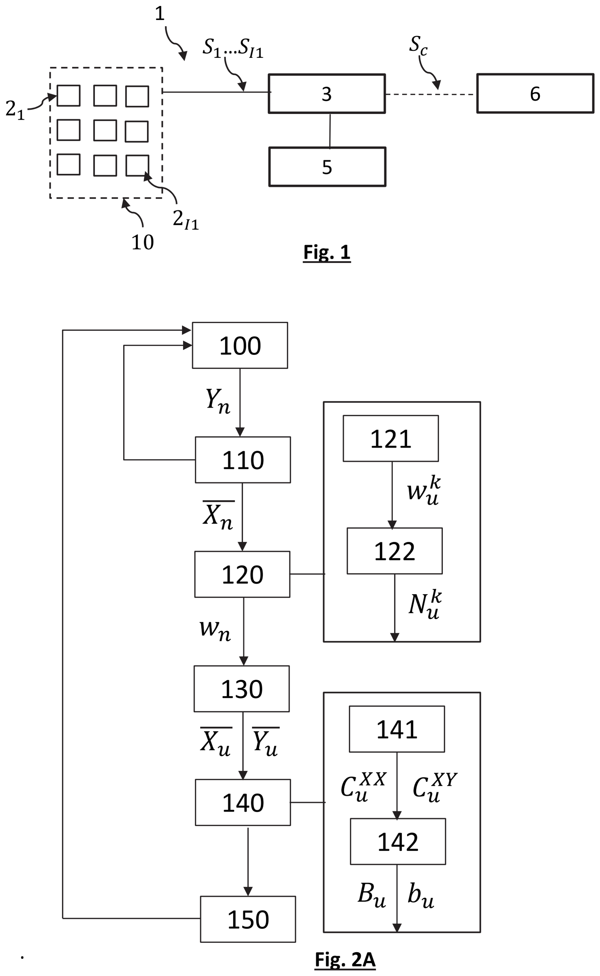

La figure 1 représente les principaux éléments d'une interface neuronale 1 selon l'invention. Il s'agit d'un dispositif comportant des capteurs $2_1...2_{I1}$, permettant l'acquisition de signaux électrophysiologiques représentatifs d'une activité neuronale. I1 est un entier correspondant au nombre de capteurs. Les capteurs $2_1...2_{I1}$ sont par exemple des électrodes corticales. Les capteurs $2_1...2_{I1}$ sont reliés à une unité de traitement 3, par exemple un microprocesseur, par une liaison filaire ou sans fil. Chaque capteur $2_1...2_{I1}$ est configuré pour détecter un signal électrophysiologique émis par un utilisateur 10. A partir de chaque signal électrophysiologique détecté, chaque capteur $2_1...2_{I1}$ transmet un signal électronique $S_1...S_{I1}$ à l'unité de traitement. L'unité de traitement 3 est apte à mettre en œuvre des algorithmes, de type modèle prédictif de commande, pour détecter des caractéristiques des signaux électrophysiologiques $S_1...S_{I1}$ spécifiques à une tâche effectuée par l'utilisateur. L'unité de traitement 3 peut par exemple être un processeur relié à une mémoire mettant en œuvre des instructions permettant d'effectuer des algorithmes de décodage tels que décrits dans les publications citées en lien avec l'art antérieur. Ces derniers permettent de décoder les signaux physiologiques détectés de façon à déterminer les caractéristiques des signaux électrophysiologiques corrélées des tâches mentales effectuées par l'utilisateur 10.

Par tâche mentale, désignée par la suite tâche, on entend une action imaginée par un utilisateur auquel l'interface neuronale directe est raccordée. Il s'agit d'une action correspondant à une intention d'effectuer une tâche spécifique. La tâche spécifique est instruite à l'utilisateur par une tierce personne ou par un algorithme dédié.

Lors du fonctionnement opérationnel de l'interface neuronale directe 1, comme mentionné en relation avec l'art antérieur, l'utilisateur réalise successivement des tâches mentales. L'unité de traitement 3 reçoit les signaux électro-physiologiques $S_1...S_{I1}$ transmis par les capteurs $2_1...2_{I1}$, représentatifs des signaux électrophysiologiques produits par l'utilisateur et détectés par les capteurs. A partir des signaux électrophysiologiques détectés, l'unité de traitement applique le modèle prédictif de commande, pour générer un signal de commande $S_c$ à l'attention d'un actionneur 6. Ainsi, l'interface neuronale directe décode les signaux électrophysiologiques produits par l'utilisateur 10 de façon à générer, à l'aide d'un modèle prédictif de commande, des signaux de commande à un actionneur. Le modèle prédictif est dit modèle prédictif de commande, car il est destiné à piloter un actionneur pour permettre d'exécuter une tâche motrice. La qualité du décodage est d'autant meilleure que l'algorithme de décodage a fait l'objet d'un apprentissage de qualité.

L'interface neuronale est également configurée pour décoder un état de satisfaction de l'utilisateur à l'égard de l'exécution de tâches qu'il a imaginé. A cet effet, l'interface peut comporter des électrodes auxiliaires 5, de type EEG ou ECoG, destinées à capter des signaux représentatifs de l'état de satisfaction de l'utilisateur. Comme décrit dans la publication Rouanne, les électrodes peuvent adresser une zone corticale adaptée à l'observation d'états de satisfaction ou d'insatisfaction de l'utilisateur. Il peut par exemple s'agir du gyrus frontal médial et moyen. Il est connu que l'activité du cortex préfrontal médian joue un rôle central dans le sentiment de satisfaction. Les électrodes auxiliaires 5 sont reliées à l'unité de traitement 3. De façon alternative, les électrodes permettant le décodage de l'état de satisfaction de l'utilisateur sont les électrodes corticales $2_1....2_{I1}$ utilisées pour décoder les intentions de l'utilisateur.

Au cours de l'apprentissage, l'utilisateur dispose d'une liste $T$ de tâches $T_k$ à effectuer. Comme décrit en lien avec l'art antérieur, au cours d'une phase d'apprentissage, on demande à l'utilisateur d'effectuer successivement des tâches $k$ choisies parmi la liste des $K$ tâches. L'objectif est de déterminer progressivement les caractéristiques électrophysiologiques les mieux corrélées avec les tâches. Ces caractéristiques permettent ensuite d'établir le modèle prédictif de commande, mis en œuvre lors du décodage, par lequel l'utilisateur 10 peut piloter l'actionneur 6 relié à l'unité de traitement 3.

Chaque tâche est à effectuer au cours d'une époque temporelle n. Le nombre d'époques à considérer pour l'apprentissage est très élevé, pouvant atteindre plusieurs centaines ou plusieurs milliers. Dans EP3985530, cité dans l'art antérieur, on décrit les principes d'un apprentissage par REW-NPLS. Selon un tel apprentissage, on dispose de données d'observation formant un tenseur tridimensionnel à chaque époque n.

Les signaux électrophysiologiques enregistrés font l'objet d'un prétraitement, selon lequel le signal de chaque électrode, durant chaque époque, fait l'objet d'une analyse fréquentielle. Il peut par exemple s'agir d'une transformée en ondelettes, par exemple une transformée en ondelette de Morlet, ou une décomposition CCWT (Continous Complex Wavelet Transform). La durée de chaque époque n peut être de 1 seconde ou 2 secondes, avec un recouvrement temporel entre deux époques consécutives. Plus précisément, durant chaque époque, une analyse fréquentielle est effectuée à intervalles réguliers, par exemple toutes les 100 ms. Une époque regroupe ainsi plusieurs analyses fréquentielles décalées dans le temps. Au cours d'une époque, plusieurs analyses fréquentielles sont effectuées, temporellement décalées les unes des autres.

A chaque époque n, on peut associer un tenseur d'observation $\overline{X_n}$, dont :

- le premier mode correspond à la position de chaque électrode, de dimension I1.
- le deuxième mode correspond aux positions temporelles des ondelettes, de dimension I2 ;
- le troisième mode correspond aux bandes de fréquences résultant de l'analyse fréquentielle, de dimension I3 ;

Une séquence d'apprentissage $u$ comporte $N$ époques n, s'étendant selon une plage temporelle $\delta t$. $u$ est un indice entier assigné chronologiquement à chaque séquence. A chaque séquence d'apprentissage u correspond un tenseur d'apprentissage $\overline{X_u}$, de dimension NxI1xI2xI3 : le tenseur d'apprentissage $\overline{X_u}$ regroupe $N$ tenseurs d'observation $X_n$. D'une façon plus générale, le tenseur d'apprentissage $\overline{X_u}$ est de dimension $N$xI1...x Ih x...IH, avec $1 \leq h \leq H$, h étant un indice et $H$ étant un entier positif. Dans cet exemple, $H = 3$.

A chaque époque n correspond un signal de commande $Y_n$, qui peut être représenté par un vecteur de commande de dimension (K,1). Chaque terme du vecteur de commande correspond à une tâche $T_k$, parmi la liste T des tâches prédéfinies. Par exemple, chaque terme du vecteur de commande est une probabilité d'exécution de la tâche $T_k$. Au cours des N époques formant la plage temporelle u, les différents signaux de commande forment une matrice $Y_u$ de dimension (K,N).

De façon alternative, le signal de commande $Y_n$ peut être une matrice, voire un tenseur multidimensionnel, auquel cas les différents signaux de commande forment un tenseur $\overline{Y_u}$ de mode $N$xJ$_1$...x J$_g$ x...J$_G$. G $\geq$ 1. D'une façon générale, chaque tâche k correspond à un signal de commande spécifique.

Lorsque l'interface est mise en œuvre, le modèle prédictif de commande F permet de passer du tenseur d'observation $\overline{X_n}$ à un signal de commande $Y_n$ pour chaque époque *n*.

Le modèle prédictif de commande F peut notamment être un modèle multilinéaire, appris par régression entre $\overline{X_u}$ et $Y_u$, par exemple par une méthode de type moindres carrés partiels multivariée (NPLS). Un tel modèle permet une estimation du signal de commande selon une expression de type : $\hat{Y}_n = F(\overline{X_n})$ où F est le modèle prédictif de commande.

$$\hat{Y}_n = B\,\overline{X_n} + b \quad (1),$$

où

- B est un tenseur de prédiction, comportant des coefficients de prédiction ;
- b est un tenseur de biais ;

Le terme tenseur regroupe à la fois un vecteur (tenseur d'ordre 1), une matrice (tenseur d'ordre 2) ou des tenseurs d'ordre supérieur.

Dans l'exemple décrit par la suite, de façon non limitative, le modèle prédictif de commande est tel que : $\hat{Y}_n = B X_n + b$ (1'), où $B$ est une matrice de dimensions *(K, P)*, $X_n$ et b sont des vecteurs de dimension (P, 1) et *(K, 1)*. $X_n$ est

un vecteur résultant d'une vectorisation du tenseur $\overline{X_n}$.avec $\quad P = \prod_{h=1}^{H} I_h$

Ainsi, l'objectif du modèle prédictif de commande est d'estimer, lors de l'utilisation de l'interface, à une époque n, le vecteur de commande $\hat{Y}_n$ selon (1) ou (1'), ce dernier traduisant l'action motrice commandée par l'utilisateur.

L'expression (1), peut être utilisée pour établir une probabilité d'émission. En prenant en compte des probabilités de changements d'états, l'état de l'utilisateur, en différents instants successifs, peut être estimé selon un modèle de Markov à états cachés (HMM), au cours duquel la tâche effectuée par l'utilisateur, en différents instants successifs, est assimilée à un état. En mettant en œuvre un algorithme, par exemple l'algorithme de type forward algorithm, on peut évaluer les différents états successifs pris par l'utilisateur. Chaque état correspond alors à l'exécution d'une tâche. Les états successifs sont estimés par un algorithme de type HMM, en utilisant le modèle prédictif de commande motrice, comme décrit dans EP3789852.

Une particularité de l'invention est que l'établissement du modèle prédictif de commande motrice, ou sa mise à jour, prend en compte un état de satisfaction de l'utilisateur, à l'aide des électrodes $2_1...2_{l1}$ ou des électrodes auxiliaires 5, comme décrit dans le brevet EP4024170 ainsi que la publication Rouanne décrits en lien avec l'art antérieur. L'état de satisfaction de l'utilisateur est estimé en appliquant un modèle de décodage, destiné à estimer l'état de satisfaction de l'utilisateur à partir de données d'observation dites auxiliaires, obtenues à partir de caractéristiques de signaux electro-physiologiques résultant des des électrodes $2_1...2_{l1}$ ou des électrodes auxiliaires 5.

On va décrire, en lien avec les figures 2A à 2C, les principales étapes d'un procédé permettant une mise en œuvre de l'invention, de façon à former un modèle prédictif de commande tel que décrit par (1) ou (1'). Sachant que le modèle prédictif de commande est élaboré en ligne, c'est-à-dire en temps réel ou quasi-temps réel, avec une mise à jour itérative, comme décrit dans EP3985530.

Pour cela, on forme des tenseurs d'observation $\overline{X_u}$ et de commande $Y_u$ au cours de séquences d'apprentissage u successives, chaque séquence d'apprentissage comportant plusieurs époques. Le modèle prédictif de commande est mis à jour en prenant en compte d'un facteur d'oubli $\lambda$, généralement compris entre 0 et 1. Le facteur d'oubli $\lambda$ est appliqué, lors de la régression, à des grandeurs résultant de la séquence précédente. Lorsque la régression est de type NPLS, le facteur d'oubli $\lambda$ est appliqué à une matrice de covariance croisée du tenseur d'apprentissage et du tenseur de commande, ainsi qu'à une matrice de covariance du tenseur d'apprentissage , comme décrit par la suite.

Dans la description qui suit, les étapes impliquant un traitement mathématique sont mises en œuvre par l'unité de traitement 3.

Les étapes présentées par la suite sont divisées en trois blocs A, B et C décrits par la suite.

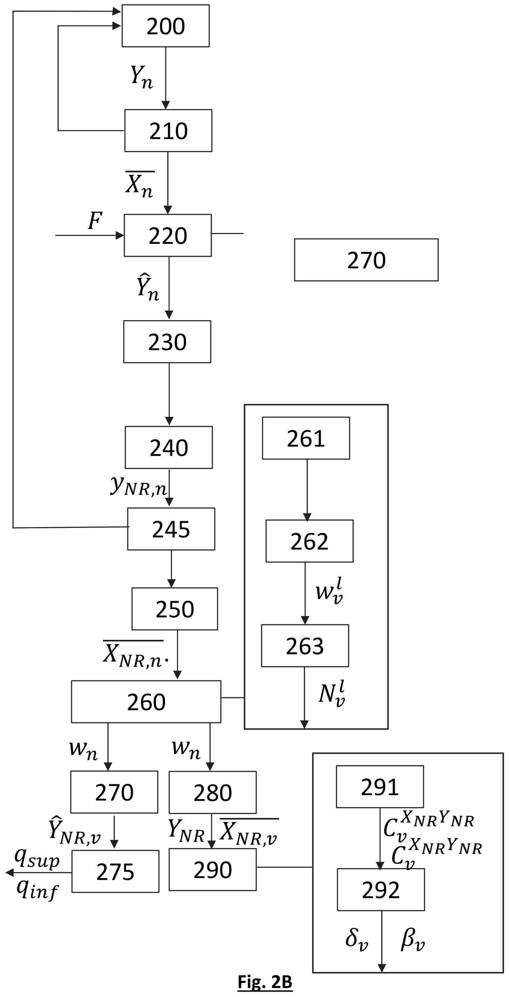

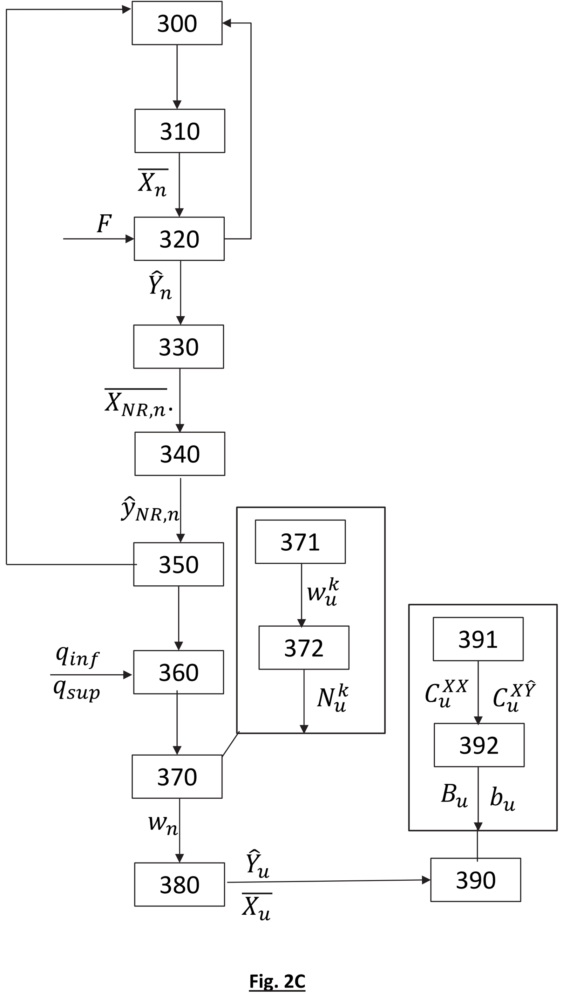

- Bloc A : apprentissage initial récursif du modèle prédictif de commande: cf. figure 2A
- Bloc B : apprentissage du modèle : cf. figure 2B
- Bloc C : mise à jour auto-adaptative et récursive du modèle prédictif de commande: cf. figure 2C

Le bloc A comporte les étapes 100 à 150. L'objectif du bloc A est d'obtenir un modèle prédictif de commande initial, c'est-à-dire un modèle de régression permettant d'estimer une intention de l'utilisateur à partir des données d'observation. Ces étapes ne sont pas nécessaires à la mise en œuvre de l'invention : le modèle prédictif de commande peut être appris d'une autre façon, par exemple hors ligne, ou déjà existant. L'invention peut également être mise en œuvre sans modèle prédictif de commande initial : dans ce cas, on affecte une probabilité d'occurrence équiprobable de chaque tâche. Cependant, l'exécution de ces tâches, durant plusieurs séquences consécutives, permet d'obtenir une initialisation du modèle prédictif de commande, ce qui améliore la performance de l'apprentissage auto-adaptatif décrit par la suite. Les étapes 100 à 150, ainsi que certaines variantes applicables, sont décrites dans la demande FR2415310 déposée le 27/12/2024.

<u>Etape 100 :</u> l'utilisateur imagine une tâche *k,* à un instant *t.* La tâche choisie à l'instant *t* peut notamment correspondre à un mouvement de l'actionneur, choisi parmi K tâches possibles. Au même instant, on enregistre les signaux électrophysiologiques résultant des différents capteurs. Plus précisément, les signaux électrophysiologiques sont enregistrés au cours d'une époque n, s'étendant selon une durée $\delta t$ à partir de l'instant t.

Chaque terme $Y_n(k)$ du vecteur de commande correspond à une probabilité d'exécution de la tâche k. Une des tâches peut être une tâche d'inactivité, désignée par l'acronyme IS (Inactive State).

<u>Etape 110 :</u> Pré-traitements. A chaque époque n, les signaux font l'objet d'une analyse temps-fréquence, comme décrit ci-avant, de façon à former un tenseur d'observation $\overline{X_n}$.

Les étapes 100 et 110 sont répétées *N* fois, de façon à former un tenseur d'apprentissage $\overline{X_u}$. *N* peut par exemple être égal à quelques dizaines ou quelque centaines. N est le nombre d'époques n formant la séquence d'apprentissage u. Par exemple, la durée totale des étapes 100 à 110 peut être de 15 secondes, chaque époque durant une durée $\delta t$ de 1 seconde, avec un décalage de 100 ms entre deux époques successives n, n+1, ce qui implique un recouvrement de 90% entre deux époques successives.

<u>Etape 120 :</u> Assignation d'un poids à chaque époque de la séquence.

Les inventeurs ont constaté qu'un apprentissage récursif selon l'art antérieur, tel que décrit EP3563218 peut conduire à un déséquilibre de classes. Par déséquilibre des classes, on entend un déséquilibre de l'occurrence de certaines classes, correspondant respectivement à certains termes du vecteur de commande. En effet, certaines tâches, correspondant respectivement à certains termes du vecteur de commande, peuvent être sous-représentées, et nécessiter une durée d'apprentissage plus longue. Par exemple, lorsque l'actionneur est un exosquelette, la commande de la translation de la main peut nécessiter davantage d'entraînement que la commande de la rotation du poignet.

En outre, le déséquilibre affectant la difficulté d'apprentissage entre les tâches peut varier au cours du temps, entre différentes séquences d'apprentissage successives.

De plus, au cours de l'apprentissage, une tâche supplémentaire, peut être ajoutée, ce qui conduit à ajouter un terme au vecteur de commande.

Au cours de l'apprentissage, l'utilisateur, ou le superviseur, ne peut pas, par lui-même, compenser le déséquilibre entre les tâches, car le fait que l'apprentissage de certaines tâches k soit plus difficile que d'autres ne peut pas être contrôlé par l'utilisateur.

Ainsi, à chaque époque n, est assigné un poids $w_n$, dont la valeur varie selon que l'on souhaite sur ou sous représenter l'observation à ladite époque n. Plus précisément, le poids $w_n$ dépend de la tâche $k$ assignée à l'époque n, parmi les $K$ tâches possibles. La tâche $k$ assignée à l'époque $n$, correspond au terme non nul du signal de commande $Y_n$. Au cours d'une même séquence $u$, les poids $w_n$ correspondant à une même tâche k, c'est-à-dire une même tâche, ont la même valeur. Ainsi, pour une même tâche $k$, le poids assigné, au cours d'une même séquence $u$ est $w_u^k$.

Sous-Etape 121 : Détermination des poids $w_n$

- on détermine le nombre d'occurrences $N_u^{maj}$ de la classe majoritaire suite à la séquence $u$, en tenant compte des séquences précédentes : $N_u^{maj} = \max_k (\lambda N_{u-1}^k + n_u^k)$ (2) , où :

$N_{u-1}^k$ est le nombre d'occurrences de chaque classe k suite à la séquence précédente u - 1 . Lors de la première séquence, $N_{u-1}^k$ est initialisé, par exemple égal à 0.

$n_u^k$ est le nombre d'occurrences de chaque classe $k$, au cours de la séquence $u$, avant la pondération.

$\lambda$ est le facteur d'oubli précédemment décrit.

- on détermine le poids $w_u^k$ assigné à chaque classe $k$, durant la séquence courante $u$

$$w_u^k = \frac{N_u^{maj} - \lambda N_{u-1}^k}{n_u^k} \ (3)$$

$$\text{et } w_u^k = 0 \text{ si } n_u^k = 0 \ (4)$$

Il est préférable que des poids trop importants ne soient pas assignés à certaines tâches, de façon à ne pas augmenter le niveau de bruit affectant la détermination du modèle prédictif de commande. Cela revient à éviter une surpondération de certaines classes k. Ainsi, on peut imposer qu'une valeur maximale $w_{max}$ soit établie . Lorsque (6) conduit à une valeur $w_u^k$ telle que $w_u^k \geq w_{max}$ , alors $w_u^k = w_{max}$ .

Après que le poids $w_u^k$ assigné à chaque classe $k$ a été défini, le poids $w_n$ associé à l'époque n est tel que $w_n = w_u^k$ , $k$ correspondant à la tâche associée à l'époque n.

Sous-étape 122 : détermination de $N_u^k$

On détermine $N_u^k$, qui correspond au nombre d'occurrences pondéré de chaque classe *k,* par :

$$N_u^k = \lambda N_{u-1}^k + w_u^k n_u^k \ (5)$$

$N_u^k$ est destiné à être utilisé lors de la mise en œuvre des expressions (2) et (3) lors de la séquence suivante u + 1.

Outre la fréquence d'occurrences des tâches, d'autres critères de pondération peuvent être pris en compte pour assigner un poids à chaque époque n :

- la performance d'apprentissage : on peut par exemple sous-pondéré les tâches pour lesquels la performance d'apprentissage est considérée comme faible. La qualité d'apprentissage peut être évaluée par un indicateur de performance de type rappel (recall), qui correspond à un ratio entre le nombre d'occurrences de tâches correctement classées sur le nombre de tâches indiquées à l'utilisateur.
- la présence d'un outlier (valeur aberrante) à l'époque considérée , auquel cas le poids peut être choisi nul : il s'agit ici d'assigner un poids en fonction de la qualité des signaux enregistrés, de façon à minimiser ou annuler l'influence de signaux considérés comme aberrants;
- la survenue d'un changement de tâche, en sous-pondérant les instants survenant juste après un changement de tâche étant sous-pondérés par rapport aux instants postérieurs : il s'agit de prendre en compte un temps de réaction de l'utilisateur, survenant à chaque changement de tâche, et au cours duquel la réponse neurologique de l'utilisateur est considérée comme transitoire.

D'une façon plus générale, un critère de pondération est défini, pour chaque époque. Il peut s'agir d'un critère de fréquence d'occurrence de la tâche effectuée à chaque époque, ou de performance d'apprentissage de la tâche choisie à chaque époque, ou un critère de qualité des signaux enregistrés à chaque époque, ou un critère temporel suite à un changement de tâche.

Etape 130 : formation du tenseur d'apprentissage $\overline{X_u}$ et d'une matrice de commande $Y_u$ la séquence u.

A chaque séquence d'apprentissage *u* correspond un tenseur d'apprentissage $\overline{X_u}$, de dimension *N*xI1xI2xI3 : le tenseur d'apprentissage $\overline{X_u}$ regroupe *N* tenseurs d'observation $\overline{X_n}$ correspondant respectivement à N époques *n*. Chaque tenseur d'observation $\overline{X_n}$ est formé de termes $(x_{n,i_1......i_H})$, où *H* est le nombre de modes du tenseur d'observation.

La formation du tenseur d'apprentissage comporte une normalisation des tenseurs d'observation $\overline{X_n}$, puis un regroupement de chaque tenseur d'observation normalisé formé pour chaque époque n de la même séquence u.

Chaque tenseur d'observation $\overline{X_n}$ est normalisé par les opérations suivantes :

$$N_u^{Tot} = \lambda N_{u-1}^{Tot} + \sum_{n=1}^{N} w_n \ (6)$$

- $N_u^{Tot}$ est une taille de la base d'apprentissage cumulée; $N_u^{Tot}$ est la taille de la base d'apprentissage accumulée depuis le début de l'apprentissage en prenant en compte les poids.
- $\lambda$ est le facteur d'oubli précédemment décrit ;
- $w_n$ est le poids associé à chaque époque n de la séquence *u ;*
- $N_{u-1}^{Tot}$ est la taille de la base d'apprentissage suite à la séquence précédente *u* - 1. Lors de la première séquence (u = 1), on prend $N_0^{Tot} = 0.$.

On calcule ensuite une moyenne $\mu_u^{Xi}$ pour chaque terme des N tenseurs d'observation, formant la séquence u avec *i* = ($i_1......i_H$) : *i* représente une coordonnée de chaque terme du tenseur d'observation et $x_{n,i}$ est chaque terme de coordonnée *i* du tenseur d'observation $\overline{X_n}$;

On calcule ensuite une somme quadratique $SS_u^{Xi}$ :

$$SS_u^{Xi} = \lambda SS_{u-1}^{Xi} + \sum_{n=1}^{N} w_n \ x_{n,i}^2 \ (8)$$

On calcule ensuite un écart type

$$\sigma_u^{Xi} = \sqrt{\frac{SS_u^{Xi} - N_u^{Tot} \mu_u^{Xi^2}}{N_u^{Tot} - 1}} \ (9)$$

Et on normalise chaque terme de chaque tenseur d'observation $\overline{X_n}$ par :

$$x_{n,i} \leftarrow \frac{x_{n,i} - \mu_u^{Xi}}{\sigma_u^i} \ (10)$$

$\leftarrow$ signifie « est remplacé par »

On procède de la même façon pour chaque vecteur de commande $Y_n$. On calcule une moyenne $\mu_u^{Yk}$ pour chaque terme des $N$ vecteurs de commande $Y_n$ pour la séquence $u$. Dans cet exemple, chaque vecteur de commande $\overline{Y_n}$ comporte $K$ termes $y_{n,k}$.

$$\mu_u^{Yk} = \frac{1}{N_u^{Tot}} (\lambda N_{u-1}^{Tot} \mu_{u-1}^{Yk} + \sum_{n=1}^N w_n \, y_{n,k} \,) \ (11)$$

$y_{n,k}$ est un terme de coordonnée $k$ du vecteur de commande $Y_n$ ;

On calcule ensuite une somme quadratique $SS_u^{Yk}$ :

$$SS_u^{Yk} = (\lambda SS_{u-1}^{Yk} + \sum_{n=1}^N w_n \, y_{n,k}^2 \,)(12)$$

On calcule ensuite un écart type

$$\sigma_u^{Yk} = \sqrt{\frac{SS_u^{Yk} - N_u^{Tot} \mu_u^{Yk^2}}{N_u^{Tot} - 1}} \ (13)$$

Et on normalise chaque terme de chaque vecteur de commande $Y_n$, pour chaque époque n, par :

$$y_{n,k} \longleftarrow \frac{y_{n,k} - \mu_u^{Yk}}{\sigma_u^{Yk}} \ (14)$$

L'étape 130 revient à normaliser chaque tenseur d'observation $\overline{X_n}$ et chaque tenseur de commande $Y_n$ en prenant en compte le poids $w_n$ associé à chaque époque $n$ de la séquence $u$. Il s'agit de calculer une moyenne temporelle et un écart type temporel, pondérés par le poids assigné à chaque époque, de chaque terme des tenseurs d'observation et du vecteur de commande. La moyenne temporelle et l'écart type temporel sont calculés pour les termes de mêmes coordonnées, en prenant en compte chaque époque n formant la séquence u.

Chaque tenseur d'observation normalisé $\overline{X_n}$ peut être exprimé sous la forme d'un vecteur d'observation $\overline{X_n}$, de dimension P, avec P = I1 x I2 x I3, suite à la vectorisation du tenseur $\overline{X_n}$, auquel cas le tenseur d'apprentissage $\overline{X_u}$ est une matrice d'apprentissage $\overline{X_u}$ formée à partir des $N$ vecteurs d'observation normalisés : $\overline{X_u} = (X_{n=1}, ... X_{n=N})^T$. La matrice d'apprentissage $\overline{X_u}$ est de dimension $(N, P)$.

On forme également une matrice de commande $Y_u$ à partir de chaque vecteur de commande normalisé. $Y_u = (Y_{n=1}, ... Y_{n=N})^T$. Dans cet exemple, $Y_u$ est de dimension $(N, K)$ car chaque tenseur de commande $Y_n$, associé à une époque n, est un vecteur de dimension $(1, K)$.

Etape 140 : établissement du modèle prédictif de commande.

On décrit à présent l'établissement du modèle prédictif de commande à partir des tenseurs normalisés résultant de l'étape 130. Il s'agit d'établir un modèle prédictif de commande permettant d'estimer le signal de commande à partir d'un tenseur d'observation, de façon que : $\hat{Y}_n = B_u X_n + b_u$ (15). $B_u$ et $b_u$ sont les paramètres du modèle prédictif de commande résultant de la séquence u.

Dans cet exemple, la régression est un régression linéaire multivariée par moindres carrés partielle (N-PLS).

Sous-étape 141

A partir de la matrice d'apprentissage $\overline{X_u}$, et de la matrice de commande $Y_u$, on calcule les matrices de covariance et de covariance croisées :

$$C_u^{XX} = X_u^T diag(W_u)X_u + \lambda C_{u-1}^{XX} \ (16)$$

et

$$C_u^{XY} = X_u^T diag(W_u)Y_u + \lambda C_{u-1}^{XY} \ (17)$$

$diag(W_u)$ est une matrice diagonale de dimension *(N,N)*. Chaque terme de $diag(W_u)$ est le poids $w_n$ assigné à l'époque n, calculé lors de l'étape 120.

Sous-étape 142 : au cours de cette sous-étape, on détermine la matrice $B_u$ et le vecteur $b_u$ à partir des matrices de covariance et de covariance croisées $C_u^{XX}$ et $C_u^{XY}$ résultant de la sous-étape précédente, comme décrit dans EP3563218. Cela correspond notamment à l'étape 140 de EP3563218. Dans EP3563218, le modèle prédictif de commande est mis à jour par régression linéaire multivariée par moindres carrés partiels (NPLS), mais d'autres types de régressions multivariées peuvent être utilisables.

Le modèle prédictif de commande peut être utilisé pour estimer la tâche la plus probable commandée par l'utilisateur, par un algorithme de type modèle de Markov caché (HMM), chaque tâche étant assimilée à un état de l'utilisateur, comme précédemment décrit.

Etape 150 : réitération. Les étapes 100 à 140 sont réitérées pour une séquence suivante, ce qui permet une mise à jour régulière, voire en continu, du modèle prédictif de commande. Le nombre d'itérations des étapes 100 à 140 est compris entre 1 et quelques dizaines ou quelques centaines. On dispose ainsi d'un modèle prédictif de commande F initialisé.

Les étapes du bloc B sont destinées à l'apprentissage supervisé du décodeur des états de satisfaction de l'utilisateur. A l'aide des signaux obtenus au niveau des électrodes, l'unité de traitement détermine, pour chaque époque, une variable scalaire $y_{NR}^n$ représentative de l'état de satisfaction ou de non satisfaction de l'utilisateur. La variable scalaire peut par exemple être comprise entre 0 et 1. L'objectif de l'apprentissage est notamment de définir, pour chaque époque :

- un seuil bas, en deçà duquel on considère que l'utilisateur n'est pas satisfait,
- un seuil haut, au-delà duquel on considère que l'utilisateur est satisfait.

Il s'agit également de mettre à jour le modèle de décodage, de façon récursive.

Les principales étapes du bloc B sont :

Etape 200 : Cette tâche est similaire à la tâche 100 précédemment décrite. L'utilisateur imagine une tâche *k*, à un instant *t*, initiant une époque n, qui correspond à un vecteur de commande $Y_n$ connu. La durée $\delta t$ de chaque époque *n* peut être d'une seconde.

Etape 210 : Pré-traitements. Cette tâche est similaire à la tâche 110, et comporte une analyse temps-fréquence des signaux enregistrés durant l'époque n. Cela permet de former un tenseur d'observation $\overline{X_n}$ pour l'époque n comme décrit en lien avec l'étape 110.

Etape 220 : mise en œuvre du modèle prédictif de commande F, défini lors du bloc A, à partir du tenseur d'observation résultant de l'étape 210. Le tenseur d'observation $\overline{X_n}$ peut être déployé de façon à former un vecteur d'observation $\overline{X_n}$, destiné à être traité par le modèle prédictif de commande de façon à estimer un signal de commande $\hat{Y}_n$ selon (1) ou (1').

Etape 230: détection d'un changement d'état du signal de commande $\hat{Y}_n$ et sélection d'époques.

Le signal de commande $\hat{Y}_n$ résultant de l'étape 220 est considéré comme représentatif, aux erreurs d'estimation près, à la tâche motrice la plus probable imaginée par l'utilisateur : il s'agit de la tâche prédite par le modèle. Un changement de tâche, entre deux époques consécutives, peut être détecté, car il correspond à un changement du signal de commande $\hat{Y}_n$. Par exemple, chaque terme du signal de commande $\hat{Y}_n$ est assigné à une tâche k, et correspond à une probabilité d'exécution de ladite tâche. Un changement de tâche correspond à un changement du terme ayant la valeur maximale dans $\hat{Y}_n$. En cas de détection d'un changement de tâche, l'étape 240 est temporisée, de façon à n'être exécutée qu'après un temps de réaction RT. Les étapes 200 à 230 sont réitérées successivement, de façon à avoir une même tâche prédite durant un nombre d'époques correspondant au temps de réaction. Le temps de réaction RT peut par exemple être de 500 ms.

Chaque époque n s'étend selon une durée $\delta t$ de 1s. Lors de la réitération des étapes 200 à 230, deux époques successives sont temporellement décalées de 100 ms. Il y a ainsi un recouvrement de 90% entre deux époques successives. Le nombre d'itérations formant une séquence est par exemple égal à 150, soit une durée de 15 secondes.

L'étape 230 revient à sélectionner temporellement au moins une époque, survenant suite à une succession d'époques (réitération des étapes 200 à 230) pour lesquelles la tâche prédite est identique. La sélection temporelle des époques permet de prendre en compte, par la suite, des époques auxquelles la réponse physiologique de l'utilisateur considérée comme stabilisée.

Les étapes suivantes sont effectuées pour chaque époque sélectionnée lors de l'étape 230.

Etape 240 : labellisation de l'époque n : un label $y_{NR,n}$ est assigné à l'époque $n$ en fonction de l'écart entre $\hat{Y}_n$ (déterminé par l'étape 220) et $Y_n$ (connu, résultant de 200). Lorsque $\hat{Y}_n$ et $Y_n$ sont considérés comme proches, $y_{NR,n}$ prend une valeur représentative d'une satisfaction de l'utilisateur. Par exemple, $y_{NR,n}$ = 1. Lorsque $\hat{Y}_n$ et $Y_n$ sont considérés comme différents, $y_{NR,n}$ prend une valeur représentative d'une insatisfaction de l'utilisateur pour la tâche k associée à l'époque n. Par exemple, $y_{NR,n}$ = 0. L'acronyme NR signifie Neural Response (réponse neuronale), ce qui correspond à l'état de satisfaction de l'utilisateur.

Etape 245 : réitérations : les étapes 200 à 240 sont réitérées et mises en œuvre pour une époque suivante n+1, jusqu'à l'atteinte d'un nombre N' d'itérations sélectionnées au cours de l'étape 230. *N'* peut par exemple être égal à quelques dizaines ou quelques centaines. Les N' époques consécutives forment une séquence d'apprentissage v.

Etape 250 : formation des tenseurs d'observation auxiliaires.

On forme, pour chaque époque sélectionnée lors de l'étape 230, un tenseur d'observation, dit tenseur d'observation auxiliaire, $X_{NR,n}$.

Pour chaque époque, le tenseur d'observation auxiliaire $\overline{X_{NR,n}}$ peut être identique au tenseur d'observation $\overline{X_n}$ formé suite à l'étape 210 pour chaque époque sélectionnée formant la séquence v. C'est notamment le cas lorsque l'on considère que les données d'observation formant le tenseur $\overline{X_n}$ peuvent être utilisées pour décoder l'état de satisfaction de l'utilisateur.

Chaque tenseur d'observation auxiliaire $\overline{X_{NR,n}}$ peut être différent des tenseurs d'observation résultant de chaque étape 210. C'est notamment le cas lorsqu'on utilise des électrodes différentes pour établir la commande motrice (étapes 200 et 210), et/ou lorsqu'on utilise des caractéristiques différentes des signaux enregistrés.

On dispose ainsi de *N'* tenseurs d'observation auxiliaires $\overline{X_{NR,n}}$ chaque tenseur d'observation auxiliaire étant différent ou égal au tenseur d'observation moteur établi lors de l'étape 210, à chaque tenseur d'observation auxiliaire correspondant un label $y_{NR,n}$.

Les étapes 260 à 290 sont destinées à effectuer ou mettre à jour, en ligne, l'apprentissage du décodeur d'état mental de l'utilisateur exécuté par l'unité de traitement 3.

Etape 260 : Définition de sous-classes et pondération de chaque sous-classe.

Sous-étape 261 : définition des sous-classes

Suite à une séquence v, on cherche à déterminer les occurrences des tâches *k*, ordonnées lors de l'étape 200, qui sont labelisées correctes ou incorrectes. On détermine autant de classes que de tâches ordonnées pendant la séquence v. Chaque classe est subdivisée en deux sous-classes *l*, de façon que :

- chaque tâche identique *k* labellisée correcte appartient à la même sous-classe ;
- chaque tâche identique *k* labellisée incorrecte appartient à la même sous-classe.

Par la suite, chaque sous-classe est désignée *l*.

Sous-Etape 262 : Assignation de poids $w_v^l$ à chaque sous-classe.

On détermine le nombre d'occurrences $N_v^{maj}$ de la classe majoritaire suite à la séquence v : , où :

- $N_{v-1}^l$ est le nombre d'occurrences de chaque sous-classe *l* suite à la séquence précédente *v* - 1 . Lors de la première séquence, $N_{v-1}^l$ est initialisé, par exemple égal à 0.

- $n_v^l$ est le nombre d'occurrences de chaque sous-classe *l*, au cours de la séquence v, avant la pondération.

Ensuite, on détermine le poids $w_v^l$ assigné à chaque sous-classe *l* par

$$w_v^l = \frac{N_v^{maj} - \lambda N_{v-1}^l}{n_v^l} \ (21)$$

Sous-étape 263 : détermination de $N_v^l$

On détermine $N_v^l$, qui correspond au nombre d'occurrences pondéré de chaque classe *l*, par :

$$N_v^l = \lambda N_{v-1}^l + w_v^l \, n_v^l \ (21')$$

$N_u^l$ est destiné à être utilisé lors de la mise en œuvre des expressions (20) et (21) lors de la séquence suivante v + 1.

Selon une possibilité, les poids associés à chaque sous-classe sont déterminés en prenant en compte un autre critère de pondération, tel que décrit en lien avec l'étape 120 : performance d'apprentissage, qualité des signaux

Suite à l'étape 260, un poids est assigné à chaque époque sélectionnée n, qui correspond au poids $w_v^l$ calculé pour la sous-classe *l* à laquelle la tâche *k* ordonnée à ladite époque, appartient <u>Etape 270</u> : Décodage de l'état de satisfaction de l'utilisateur.

Il s'agit d'utiliser tout ou partie des signaux résultant des électrodes pour estimer, un état de satisfaction $\hat{y}_{NR,n}$ de l'utilisateur relativement au signal de commande $\hat{Y}_n$. On met en œuvre le modèle de décodage à partir du tenseur d'observation auxiliaire $\overline{X_{NR,n}}$ correspondant à chaque époque sélectionnée n, de façon à obtenir un état de satisfaction $\hat{y}_{NR,n}$.

On forme également un vecteur de décodage $\hat{Y}_{NR,v} = (\hat{y}_{NR,n=1} \ldots\ldots \hat{y}_{NR,n=N'})^T$ résultant des états de satisfaction $\hat{y}_{NR}^n$ estimés pour chaque époque prise en compte lors de l'étape 240.

$$\hat{Y}_{NR,v} \ \in \mathbb{R}^{N'}$$

<u>Etape 275</u> : Définition de seuils

On trace un histogramme des valeurs de $\hat{y}_{NR,n}$ respectivement pondérées par le poids $w_n$ associé à la sous-classe *l* associée à l'époque *n*. Cela revient à obtenir l'histogramme, dit histogramme pondéré, des valeurs pondérées $w_n \hat{y}_{NR,n}$.

A partir de l'histogramme pondéré, on détermine des seuils correspondant à des fractiles de la distribution des valeurs $w_n \hat{y}_{NR,n}$. On prend un compte :

- un fractile inférieur, par exemple un fractile à 10%, délimitant les 10% de valeurs les plus faibles de l'histogramme. Le fractile inférieur définit un seuil inférieur $q_{inf}$ de façon que par la suite, chaque valeur de $\hat{y}_{NR,n}$ déterminée par le décodeur de satisfaction, inférieure à $q_{inf}$, est considérée comme un état d'insatisfaction.
- et un fractile supérieur, par exemple un fractile à 90%, par exemple un fractile à 90%. Le fractile supérieur définit un seuil inférieur $q_{sup}$ de façon que par la suite, chaque valeur de $\hat{y}_{NR,n}$ déterminée par le décodeur de satisfaction, supérieure à $q_{sup}$, est considérée comme un état de satisfaction.

La détermination du seuil inférieur et du seuil supérieur, conditionnant les états de satisfaction et d'insatisfaction, est ainsi effectuée à partir de l'histogramme pondéré.

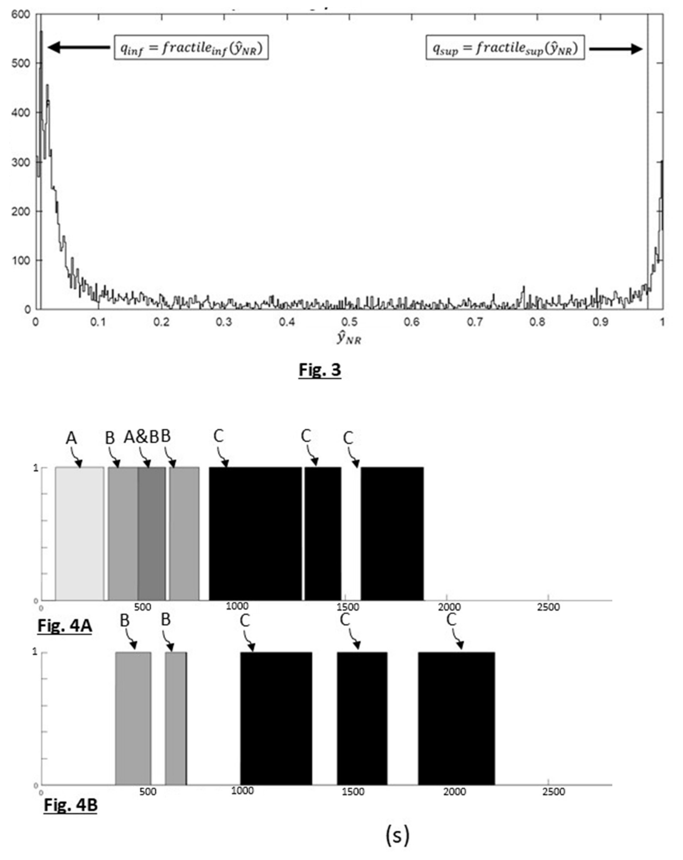

La figure 3 représente un exemple d'histogramme pondéré, sur lequel on a représenté un seuil inférieur et un seuil supérieur. Sur la figure 3, le fractile à 10% est désigné *fractile*$_{inf}$ et le fractile à 90% est désigné *fractile*$_{sup}$. L'axe des ordonnées correspond au nombre d'époques.

Au cours du bloc B, on peut également mettre à jour le modèle de décodage de l'état de satisfaction de l'utilisateur. Pour cela, on dispose, pour les *N'* époques sélectionnées dans l'étape 230, et pour lesquelles on dispose du vecteur de labels $Y_{NR,v}$ formé par l'ensemble des labels $y_{NR,n}$ déterminés lors de chaque étape 240. $Y_{NR,v} = (y_{NR,n=1} \ldots\ldots y_{NR,n=N'})^T$

Le modèle de décodage de l'état de l'utilisateur peut être mis à jour effectuant une régression entre le vecteur de labels $Y_{NR,v}$ et un tenseur d'apprentissage $\overline{X_v}$, formé à partir des tenseurs d'observation auxiliaire $X_{NR,n}$.

De préférence, la mise à jour du modèle de décodage prend en compte les poids définis lors de l'étape 260.

Cela permet une mise à jour du décodeur d'état de satisfaction de l'utilisateur en utilisant un faible nombre de données. Cela permet une mise à jour simple et rapide du décodeur, sans mobilisation de ressources mémoire importantes ; Le modèle de décodage peut être mis à jour régulièrement, par exemple tous les mois, ou tous les 3 ou 6 mois.

La mise à jour du modèle de décodage correspond aux étapes suivantes :

Etape 280 : Formation du tenseur d'apprentissage $\overline{X}_{NR,v}$ et du vecteur de labels $Y_{NR,v}$.

Chaque tenseur d'observation auxiliaire $\overline{X}_{NR,n}$, et le vecteur $Y_{NR,v}$ sont normalisés par les opérations suivantes.

$$N_v^{Tot} = \lambda N_{v-1}^{Tot} + \sum_{n=1}^{N'} w_n \ (22)$$

- $N_v^{Tot}$ est une taille de la base d'apprentissage cumulé depuis le début de l'apprentissage ;
- $\lambda$ est le facteur d'oubli précédemment décrit ;
- $w_n$ est le poids associé à chaque époque n de la séquence v : le poids de chaque époque correspond à la sous-classe *l* assignée à l'époque.
- $N_{v-1}^{Tot}$ est le terme de normalisation pour la séquence précédente v - 1. Lors de la première séquence de décodage (v = 1), on prend $N_0^{Tot} = 0$.

On calcule ensuite une moyenne $\mu_v^{X_{NR,i}}$ pour chaque terme des $N'$ tenseurs d'observation auxiliaire $\overline{X_{NR,n}}$ formant la séquence v.

-

$$\mu_v^{X_{NR,i}} = \frac{1}{N_v^{Tot}} (\lambda N_{v-1}^{Tot} \mu_{v-1}^{X_{NR,i}} + \sum_{n=1}^{N'} w_n \, x_{NR,n,i} )(23)$$

avec $i = (i_1 ...... i_H)$ : *i* représente une coordonnée de chaque terme du tenseur d'observation et $x_{NR,n,i}$ est chaque terme de coordonnée i du tenseur d'observation auxiliaire $X_{n,NR}$ de la séquence v;

- On calcule ensuite une somme quadratique $SS_v^{Xi}$ :

$$SS_v^{Xi} =$$

$$(\lambda SS_{v-1}^{Xi} + \sum_{n=1}^{N'} w_n \, x_{NR,n,i}^2 )(24)$$

- On calcule ensuite un écart type

$$\sigma_v^{Xi} = \sqrt{\frac{SS_v^{Xi} - N_v^{Tot} \mu_v^{X_{NR,i}2}}{N_v^{Tot} - 1}} \ (25)$$

- Et on normalise chaque terme $x_{n,NR,i}$ de chaque tenseur d'observation $\overline{X_{NR,n}}$ par :

-

$$x_{NR,n,i} \leftarrow \frac{x_{n,NR,i} - \mu_v^{X_{NR,i}}}{\sigma_v^i} \ (26)$$

On forme un vecteur de labels. Le vecteur de labels $Y_{NR,v}$ est formé par l'ensemble des labels $y_{NR,n}$ déterminés lors de chaque étape 240. Ainsi :

-

$$Y_{NR,v} = (y_{NR,n=1} \cdots y_{NR,n=N'})^T$$

-

$$Y_{NR,v} \in \mathbb{R}^{N' \times 2}$$

On calcule ensuite une moyenne $\mu_v^{Y_{NR}}$ pour chaque terme des $N'$ labels $y_{NR,n=1} \ldots\ldots y_{NR,n=N'}$

$$\mu_v^{Y_{NR}} = \frac{1}{N_v^{Tot}}(\lambda N_{v-1}^{Tot}\mu_{v-1}^{Y_{NR}} + \sum_{n=1}^{N'} w_n\, y_{NR,n}) \quad (27)$$

On calcule ensuite les sommes quadratiques :

$$SS_v^{Y_{NR}} = \lambda SS_{v-1}^{Y_{NR}} + \sum_{n=1}^{N'} w_n\, (y_{NR,n})^2 (28)$$

On calcule ensuite les écarts types

$$\sigma_v^{Y_{NR}} = \sqrt{\frac{SS_v^{Y_{NR}} - N_v^{Tot}\mu_v^{Y_{NR}\,2}}{N_v^{Tot}-1}} \quad (29)$$

Et on normalise chaque terme du vecteurs de labels $Y_{NR}$

$$y_{NR,n} \leftarrow \frac{y_{NR,n} - \mu_u^{Y_{NR}}}{\sigma_v^{Y_{NR}}} \quad (30)$$

L'étape 280 revient à normaliser chaque tenseur d'observation auxiliaire $\overline{X_{NR,n}}$ et du vecteur de labels $Y_{NR}$ en prenant en compte le poids $w_n$ associé à chaque époque $n$ de la séquence $v$. Il s'agit de calculer une moyenne temporelle et un écart type temporel, pondérés par le poids assigné à chaque époque, de chaque terme des tenseurs d'observation auxiliaire et du vecteur de labels. La moyenne temporelle et l'écart type temporel sont calculés pour les termes de mêmes coordonnées, en prenant en compte chaque époque n formant la séquence v.

Chaque tenseur d'observation auxiliaire normalisé $\overline{X_{NR,n}}$ peut être exprimé sous la forme d'un vecteur d'observation auxiliaire $\overline{X_{NR,n}}$ suite à la vectorisation de chaque tenseur $X_{NR,n}$, auquel cas le tenseur d'apprentissage $\overline{X_{NR,v}}$ est une matrice d'apprentissage $\overline{X_{NR,v}}$ formée à partir des $N'$ vecteurs d'observation auxiliaires : $X_{NR,v} = (X_{NR,n=1}, \ldots X_{NR,n=Nr})^T$. La matrice d'apprentissage $\overline{X_{NR,v}}$ est de dimension $(N',P')$, où P' est la dimension de chaque vecteur d'observation auxiliaire normalisé $X_{NR,n}$.

Etape 290 : mise à jour du modèle de décodage

On décrit à présent l'établissement du modèle de décodage à partir du tenseur d'apprentissage $X_{NR,v}$ et du vecteur de labels $Y_{NR}$ résultant de l'étape 280.

Sous-étape 291

On calcule les matrices de covariance et de covariance croisées sont explicitées selon :

$$C_v^{X_{NR}\,Y_{NR}} = X_{NR,v}^T\, diag(W_v)Y_{NR,v} + \lambda C_{v-1}^{X_{NR}\,Y_{NR}} \quad (31)$$

et

$$C_v^{X_{NR}\,X_{NR}} = X_{NR,v}^T\, diag(W_v)X_{NR,v} + \lambda C_{v-1}^{X_{NR}\,X_{NR}} \quad (32)$$

$diag(W_v)$ est une matrice diagonale de dimension $(N',N')$. Chaque terme de $diag(W_v)$ est le poids $w_n$ assigné à l'époque n, calculé lors de l'étape 260.

Les matrices de covariance et de covariance croisée sont utilisées pour établir un modèle de décodage selon les

sous-étapes suivantes

<u>Sous-étape 292</u> : au cours de cette sous-étape, on détermine le modèle de décodage à partir des tenseurs $C_v^{X_{NR} Y_{NR}}$ et $C_v^{X_{NR} X_{NR}}$ . Il s'agit d'établir un modèle de décodage permettant d'estimer le signal de commande à partir d'un tenseur d'observation, de façon que :

$$\hat{Y}_{NR,n} = \beta_v \, \overline{X}_{RN,n} + \delta_v \; (33)$$

$\beta_v$ et $\delta_v$ sont les paramètres du modèle de décodage résultant de la séquence v.

Le bloc B permet une mise à jour en ligne du décodeur d'états de satisfaction, en prenant en compte un équilibrage des classes. Il en résulte une meilleure performance de décodage.

<u>Bloc C : mise à jour itérative du modèle prédictif de commande F.</u>

La complétion du bloc B et l'utilisation d'un modèle prédictif de commande initial, par exemple résultant du bloc A, permet une mise à jour dudit modèle prédictif de commande de façon non supervisée. L'intérêt est que la labellisation de chaque époque résulte du décodeur d'état de satisfaction de l'utilisateur, et non d'annotations générées par un superviseur. Le bloc C comporte les étapes suivantes :

<u>Etape 300</u> : l'utilisateur imagine la réalisation d'une tâche *k,* à un instant *t* comme décrit lors de l'étape 100. On enregistre les signaux neuronaux produits par les électrodes $2_1...2_{I1}$ au cours d'une époque n s'étendant entre *t* et *t + δt.*

<u>Etape 310</u> : les signaux neuronaux correspondant à l'époque n sont centrés et réduits. Ils font l'objet d'une analyse temps-fréquence, comme décrit en lien avec l'étape 110, de façon à former un tenseur d'observation $\overline{X}_n$.

<u>Etape 320</u> : On applique le modèle prédictif de commande F, tel que décrit en lien avec l'étape 220, au tenseur d'observation, ce dernier étant notamment déployé, pour former un vecteur d'observation $X_n$. On obtient ainsi un signal de commande $\hat{Y}_n = F(X_n)$. Dans cet exemple, chaque terme du signal de commande correspond à une probabilité qu'une tâche k soit imaginée par l'utilisateur.

Les étapes 300 à 320 sont réitérées pour plusieurs époques jusqu'à l'atteinte de la condition explicitée dans l'étape 330.

<u>Etape 330</u> : Après une durée $t + δt + RT$ à la suite d'un changement de terme maximal du vecteur $\hat{Y}_n$, si le terme maximal n'a pas changé au cours de cette durée, on enregistre les signaux neuronaux destinés à prévoir la satisfaction de l'utilisateur. Cela signifie que la tâche prédite est identique pour les époques s'étendant entre t et $t + δt + RT$. Les signaux neuronaux enregistrés forment un tenseur d'observation auxiliaire $X_{NR,n}$. Il est rappelé que le tenseur d'observation auxiliaire peut être différent ou identique du tenseur d'observation $\overline{X}_n$.

Dans l'exemple décrit par la suite, les tenseurs d'observation $\overline{X}_n$. et d'observation auxiliaires $\overline{X}_{NR,n}$ formés à chaque époque sont identiques.

<u>Etape 340</u> : On estime l'état de satisfaction de l'utilisateur $\hat{y}_{NR,n}$ en appliquant le modèle de décodage, résultant du bloc B, au tenseur d'observation auxiliaire $\overline{X}_{NR,n}$ formé dans l'étape 330.

<u>Etape 350</u> : Les étapes 300 à 340 sont répétées M fois, M peut par exemple être égal à quelques dizaines ou quelque centaines. M est le nombre d'époques n formant la séquence d'apprentissage u. Par exemple, la durée totale de la séquence peut être de 15 secondes, chaque époque durant une durée $δt$ de 1 seconde, avec un décalage de 100 ms entre deux époques successives, ce qui implique un recouvrement de 90% entre deux époques successives. M peut être égal ou différent du nombre d'époques *N* décrites dans le bloc A.

<u>Etape 360</u> : traitement des tenseurs d'observation $\overline{X}_n$ en fonction de la satisfaction de l'utilisateur. Au cours de cette étape :

- les tenseurs d'observation $\overline{X}_n$ pour lesquels $\hat{y}_{NR,n} \geq q_{sup}$ sont conservés : on considère que la tâche k correspondant à l'époque n correspond au terme maximal du vecteur $\hat{Y}_n$ ;
- les tenseurs d'observation $\overline{X}_n$ pour lesquels $q_{inf} < \hat{y}_{NR,n} < q_{sup}$ sont rejetés ;
- les tenseurs d'observation $\overline{X}_n$ pour lesquels $\hat{y}_{NR,n} \leq q_{inf}$ sont conservés : on considère que la tâche *k* associée à l'époque *n* correspond non pas au terme maximal du vecteur $\hat{Y}_n$, mais au deuxième maximum, c'est-à-dire à la tâche pour laquelle la probabilité est la plus proche de la probabilité maximale. Ainsi, pour les époques n auxquelles $\hat{y}_{NR,n} \leq q_{inf}$, le terme maximal de $\hat{Y}_n$ est mis à zéro, de façon que ce soit le deuxième maximum qui prévale suite à la mise à zéro du terme maximal.

<u>Etape 370</u> : pondération

Ainsi, à chaque époque n, est assigné un poids $w_n$, dont la valeur varie selon que l'on souhaite sur ou sous représenter l'observation à ladite époque n. Plus précisément, le poids $w_n$ dépend de la tâche $k$ assignée à l'époque n, parmi les $K$ tâches possibles. La tâche $k$ assignée à l'époque $n$, correspond au terme non nul du signal de commande $Y_n$. Au cours d'une même séquence $u$, les poids $w_n$ correspondant à une même tâche k, c'est-à-dire une même tâche, ont la même valeur. Ainsi, pour une même tâche $k$, le poids assigné, au cours d'une même séquence $u$ est $w_u^k$.

Sous-Etape 371 : Détermination des poids $\underline{w_n}$

- on détermine le nombre d'occurrences $N_u^{maj}$ de la classe majoritaire suite à la séquence $u$ :

$$N_u^{maj} = \max_k( \lambda N_{u-1}^k + n_u^k) \ (34), \text{ où :}$$

$N_{u-1}^k$ est le nombre d'occurrences de chaque classe k suite à la séquence précédente u - 1 . Lors de la première séquence, $N_{u-1}^k$ est initialisé, par exemple égal à 0. $n_u^k$ est le nombre d'occurrences de chaque classe $k$, au cours de la séquence $u$, avant la pondération.

$\lambda$ est le facteur d'oubli précédemment décrit.

- on détermine le poids $w_u^k$ assigné à chaque classe k, durant la par

$$w_u^k = \frac{N_u^{maj} - \lambda N_{u-1}^k}{n_u^k} \ (35)$$

$$\text{et } w_u^k = 0 \text{ si } n_u^k = 0 \ (36)$$

Il est préférable que des poids trop importants ne soient pas assignés à certaines tâches, de façon à ne pas augmenter le niveau de bruit affectant la détermination du modèle prédictif de commande. Cela revient à éviter une surpondération de certaines classes k. Ainsi, on peut imposer qu'une valeur maximale $w_{max}$ soit établie . Lorsque (36) conduit à une valeur $w_u^k$ telle que $w_u^k \geq w_{max}$ , alors $w_u^k = w_{max}$ .

Après que le poids $w_u^k$ assigné à chaque classe $k$ a été défini, le poids $w_n$ associé à l'époque n est tel que $w_n = w_u^k$ , k correspondant à la tâche associée à l'époque n.

Sous-étape 372 : détermination de $N_u^k$

On détermine $N_u^k$ , qui correspond au nombre d'occurrences pondéré de la classe $k$, par :

$$N_u^k = \lambda N_{u-1}^k + w_u^k n_u^k \ (37).$$

$N_u^k$ est destiné à être utilisé lors de la mise en œuvre des expressions (35) et (36) lors de la séquence suivante u + 1.

Outre la fréquence d'occurrences des tâches, d'autres critères de pondération peuvent être pris en compte pour assigner un poids à chaque époque n, comme décrit en lien avec le bloc A, Cf sous-étape 121.

Etape 380 : Formation du tenseur d'apprentissage $\overline{X_u}$ et de la matrice de commande $\hat{Y}_u$.

Chaque tenseur d'observation $\overline{X_n}$ est normalisé par les opérations suivantes :

$$N_u^{Tot} = \lambda N_{u-1}^{Tot} + \sum_{n=1}^N w_n \ (38)$$

- $N_u^{Tot}$ est une taille de la base d'apprentissage cumulée;

- $\lambda$ est un facteur d'oubli compris entre 0 et 1.
- $w_n$ est le poids associé à chaque époque n de la séquence *u ;*
- $N_{u-1}^{Tot}$ est le terme de normalisation pour la séquence précédente *u* - 1.Lors de la première séquence (u = 1), on prend $N_0^{Tot} = 0.$.

On calcule ensuite une moyenne $\mu_u^{Xi}$ pour chaque terme des N tenseurs d'observation, formant la séquence u :

$$\mu_u^{Xi} = \frac{1}{N_u^{Tot}}(\lambda N_{u-1}^{Tot}\mu_{u-1}^{Xi} + \sum_{n=1}^N w_n x_{n,i})(39) \text{ avec } i = (i_1 \ldots \ldots i_H)$$

$x_{n,i}$ est chaque terme de coordonnée *i* du tenseur d'observation $\overline{X_n}$ ;

On calcule ensuite une somme quadratique $SS_u^{Xi}$ :

$$SS_u^{Xi} = \lambda SS_{u-1}^{Xi} + \sum_{n=1}^N w_n x_{n,i}^2 \ (40)$$

On calcule ensuite un écart type

$$\sigma_u^{Xi} = \sqrt{\frac{SS_u^{Xi} - N_u^{Tot}\mu_u^{Xi^2}}{N_u^{Tot} - 1}} \ (41)$$

Et on normalise chaque terme du tenseur d'observation par :

$$x_{n,i} \leftarrow \frac{x_{n,i} - \mu_u^{Xi}}{\sigma_u^i} \ (42)$$

On procède de la même façon pour chaque vecteur de commande $\hat{Y}_n$.

On calcule une moyenne $\mu_u^{\hat{Y}k}$ pour chaque terme k des des *M'* vecteurs de commande $\hat{Y}_n$ pour la séquence u :

$$\mu_u^{\hat{Y}k} = \frac{1}{N_u^{Tot}}(\lambda N_{u-1}^{Tot}\mu_{u-1}^{\hat{Y}k} + \sum_{n=1}^N w_n \hat{y}_{n=k})(43)$$

$\hat{y}_{n=k}$ est un terme de coordonnée k de chaque vecteur $\hat{Y}_n$
On calcule ensuite une somme quadratique

$$SS_u^{\hat{Y}k} = \lambda SS_{u-1}^{\hat{Y}k} + \sum_{n=1}^N w_n \hat{y}_{n=k}^2 \ (44)$$

On calcule ensuite un écart type

$$\sigma_u^{\hat{Y}k} = \sqrt{\frac{SS_u^{\hat{Y}k} - N_u^{Tot}\mu_u^{\hat{Y}k^2}}{N_u^{Tot} - 1}} \ (45)$$

Et on normalise chaque terme de $\hat{Y}_n$ par :

$$y_{n,k} \leftarrow \frac{y_{n,k} - \mu_u^{\hat{Y}k}}{\sigma_u^{\hat{Y}k}} \ (46)$$

Chaque tenseur d'observation normalisé $\overline{X_n}$ peut être exprimé sous la forme d'un vecteur d'observation $\overline{X_n}$, de dimension P, avec P = I1 x I2 x I3, suite à la vectorisation de chaque tenseur d'observation $\overline{X_u}$ sélectionné auquel cas le tenseur d'observation normalisé est une matrice d'apprentissage $\overline{X_u}$ formée à partir des M' vecteurs d'observation normalisés : $\overline{X_u} = (X_{n=1}, ... X_{n=M'})^T$. La matrice d'apprentissage $\overline{X_u}$ est de dimension (M', P). M' correspond au nombre de tenseurs $\overline{X_n}$ sélectionnés lors de l'étape 360. $M' \leq M$.

On forme également une matrice de commande $\hat{Y}_u$ à partir de chaque vecteur de commande normalisé : $\hat{Y}_u = (\hat{Y}_{n=1}, ... \hat{Y}_{n=M'})^T$. Dans cet exemple, $\hat{Y}_u$ est de dimension (M', K).

Etape 390 : établissement du modèle prédictif de commande.

On décrit à présent l'établissement d'un modèle prédictif de commande à partir des tenseurs résultant de l'étape 380. Il s'agit d'établir, par régression, un modèle prédictif de commande permettant d'estimer le signal de commande à partir d'un tenseur d'observation, de façon que :

$$\hat{Y}_n = B_u\, X_n + b_u \ (47)$$

Dans cet exemple, la régression est un régression linéaire multivariée par moindres carrés partielle (N-PLS).

Sous-Etape 391 : formation des matrices de covariance

De façon analogue à ce qui a été décrit en lien avec (16) ou (17), à partir de $\overline{X_u}$ et de $\hat{Y}_u$, on forme les matrices de covariance et de covariance croisées. Ces dernières sont explicitées selon :

$$C_u^{XX} = X_u^T diag(W_u) X_u + \lambda C_{u-1}^{XX} \ (48)$$

et

$$C_u^{X\hat{Y}} = X_u^T diag(W_u)\hat{Y}_u \quad + \lambda C_{u-1}^{X\hat{Y}} \ (49)$$

$diag(W_u)$ est une matrice diagonale de dimension (M',M'). Chaque terme de $diag(W_u)$ est un poids $w_n$ assigné à l'époque n, dont la valeur varie en fonction de la classe k assignée à l'époque n, selon que l'on souhaite sur ou sous représenter l'observation à ladite époque n. Au cours d'une même séquence u, les poids $w_n$ correspondant à une même classe k, c'est-à-dire une même tâche, ont la même valeur

Sous-étape 392 : au cours de cette étape, on détermine la matrice $B_u$ et le vecteur $b_u$ à partir des matrices de covariance et de covariance croisées $C_u^{XX}$ et $C_u^{X\hat{Y}}$ résultant de la sous-étape précédente, de façon analogue à ce qui a été décrit dans l'étape 142 du bloc A.

Essais expérimentaux

On a mis en œuvre les étapes précédemment décrites, en enregistrant des signaux ECoG d'un utilisateur à l'aide d'un implant sans fil WIMAGINE, tel que décrit dans Mestais C. et al « WIMAGINE : Wireless 64-Channel ECoG recording implant for long term clinical applications », IEEE Transactions on neural systems and rehabilitation engineering, Vol. 23, No1, January 2015.

Lors des essais, l'utilisateur contrôlait un environnement virtuel en étant placé devant un avatar fournissant des informations visuelles au patient. L'avatar était une image miroir de l'utilisateur. La tâche demandée était indiquée sur l'avatar par un cercle orange placé au niveau de l'état de mouvement correspondant. La probabilité de prédiction de chaque tâche était indiquée sur l'avatar sous la forme d'anneaux orange dont le diamètre dépend de la probabilité des états décodés. Lors des essais en mode supervisé, Un anneau vert matérialisait le fait que la probabilité la plus élevée était cohérente avec la tâche imaginée par l'utilisateur. L'avatar disposait de 5 états possibles, en plus de l'état de repos, les mouvements suivants :

- saisie de la main droite;
- saisie de la main gauche ;
- flexion du coude droit
- flexion du coude gauche.

L'utilisateur a été soumis à un processus d'imagerie motrice directe, selon laquelle il devait imaginer effectuer l'un de ces mouvements, et de visualiser l'avatar les effectuer à l'écran. Deux séances ont été effectuées, de durées respectives 32 minutes et 53 minutes, sur une durée de 2 jours.

Au cours de chaque séance, on recevait les signaux neuronaux de 64 électrodes, avec une analyse temps-fréquence effectuée selon des époques de durée 1 seconde, deux époques successives étant décalées de 100 ms, soit un taux de recouvrement de 90%. L'analyse temps-fréquence a été mise en œuvre en utilisant la transformée en ondelettes de Morlet, avec 15 fréquences centrales espacées de 10 Hz entre 10 Hz et 150 Hz.

Durant la première séance, on a mis en œuvre un pré-apprentissage supervisé du modèle prédictif de commande, en prenant en compte 2 ou 3 instructions pour chaque état, comme décrit en lien avec le bloc A. L'avantage du pré-apprentissage du modèle prédictif de commande est d'obtenir, lors de la mise en œuvre de l'apprentissage non supervisé en ligne (bloc C), un taux raisonnable de réponses correctes et de réponses erronées. Le pré-apprentissage est suffisamment court pour pouvoir apprécier l'apprentissage non supervisé du modèle prédictif de command. L'objectif du pré-apprentissage est donc de favoriser une convergence de l'apprentissage non supervisé. Il s'agit d'obtenir un taux raisonnable de réponses correctes et de réponses erronées. Le pré-apprentissage permet ainsi un apprentissage du décodeur de l'état de satisfaction plus rapide grâce à une meilleure distribution des sous-classes d'état de satisfaction. Sans pré-apprentissage, la distribution des sous-classes d'état de satisfaction serait très orientée en faveur de réponses erronées (i.e. d'insatisfactions) et donc le décodeur de l'état de satisfaction mettrait beaucoup plus de temps pour converger vers une solution viable en terme de performances. Ainsi, L'avantage du pré-apprentissage du modèle prédictif de commande est d'autre part d'accélérer la convergence de l'apprentissage non supervisé en ligne du modèle prédictif de commande.

Suite au pré-apprentissage, un apprentissage du décodeur de l'état de satisfaction de l'utilisateur a été mis en œuvre. L'utilisateur disposait de 64 électrodes ECoG disposées au niveau du cortex sensorimoteur de l'utilisateur. L'apprentissage du décodeur a été mis en œuvre en prenant en compte des fractiles inférieur et supérieur respectivement égaux à 10% et 90%.

On a ensuite procédé à des essais de mise à jour du modèle prédictif de commande de façon non supervisée, comme décrit en lien avec le bloc C. Les états étaient indiqués à l'utilisateur, afin de mesurer la performance de décodage, mais ils n'étaient pas utilisés en tant que labels pour chaque époque.

Les figures 4A et 4B représentent la chronologie des deux séances : mise en œuvre de chaque bloc en fonction du temps (axe des abscisses). Sur les figures 4A et 4B, on a indiqué les blocs mis en œuvre en fonction du temps durant chaque séance. Le modèle prédictif de commande et le décodeur de satisfaction appris lors de la première séance ont été utilisés lors de la deuxième séance.

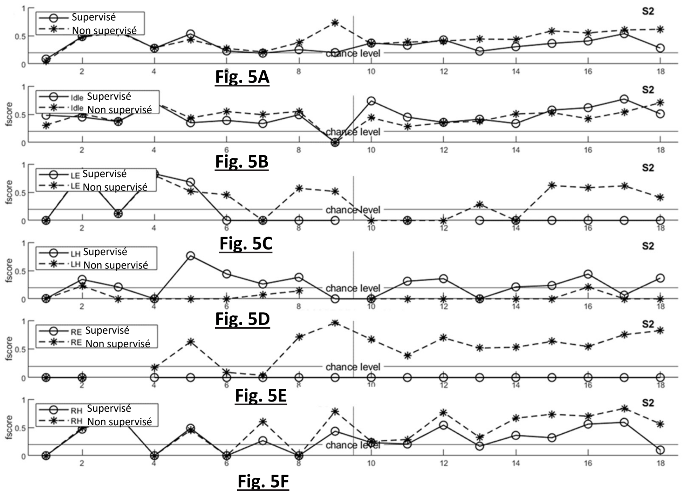

Les figures 5A à 5F montrent l'évolution des performances de l'apprentissage auto-adaptatif non supervisé. On a décomposé l'apprentissage en différents blocs d'entraînement, chaque bloc d'entraînement correspondant à une mise à jour du modèle prédictif de commande, c'est-à-dire 15 secondes d'apprentissage. Chaque bloc d'entraînement a été assigné d'un indice temporel chronologique, qui correspond à l'axe des abscisses.

Les performances de l'apprentissage ont été quantifiées en calculant deux indicateurs :

- la précision globale, ou « balanced accuracy », définie selon le ratio $\frac{\frac{TP}{TP+FN}+\frac{TN}{TN+FP}}{2}$ où TP, TN, FP et FN désignent respectivement les nombres de vrai positif, vrai négatif, faux positif, faux négatif. Le score maximal de précision global est égal à 1.

- un score, désigné fscore, défini selon le ratio $\frac{TP}{TP+\frac{FP+FN}{2}}$

Sur la figure 5A, on a représenté l'évolution de la précision globale (balanced accuracy). Sur les figures 5B à 5F, on a représenté l'évolution du fscore pour chaque état : repos (Idle), flexion du coude gauche (LE), saisie de la main gauche (LH), flexion du coude droit (RE), saisie de la main droite (RH). Sur chaque figure, on a représenté les performances obtenues :

- de manière supervisée (Symbole ∘), puis n'étant pas mis à jour par la suite : blocs A et A&B ;
- de manière non supervisée (Symbole *) (blocs C uniquement, après un pré-apprentissage (bloc A et A&B).

Sur chacune de ces figures, on a également représenté, par une ligne en pointillés, un niveau dit de chance (chance level), déterminé en prenant en compte un niveau de performance lorsque la sortie du décodeur est aléatoire.

La figure 5A permet d'apprécier le gain apporté par l'apprentissage non supervisé par rapport à l'apprentissage supervisé : la mise à jour non supervisée du modèle prédictif de commande permet d'obtenir de meilleurs performances. Les figures 5B à 5F permettent d'apprécier le gain apporté par l'apprentissage non supervisé par rapport à l'apprentissage supervisé pour 3 des 5 états, en l'occurrence la flexion du coude droit (RE : cf. figure 5E), la saisie de la main droite (RH - cf. figure 5F), et la flexion du coude gauche (LE : cf. figure 5C). Les tâches LE et RE ont fait l'objet d'un apprentissage initial volontairement limité, de façon à pouvoir apprécier l'amélioration de l'apprentissage de façon non supervisée.

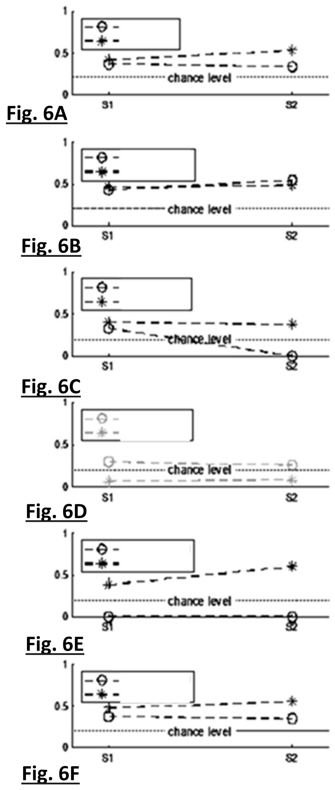

Les figures 6A à 6F montrent l'évolution des indicateurs respectivement discutés en lien avec les figures 5A à 5F, entre la séance 1 « S1 » et la séance 2 « S2 », telles que décrites en lien avec les figures 4A et 4B. Sur chaque figure, on a représenté les indicateurs obtenus par apprentissage supervisé (bloc A et A&B - symbole o) et par apprentissage non supervisé (blocs C - symbole *).

La figure 6A correspond à la précision globale. Sur les figures 6B à 6F, on a représenté l'évolution du fscore pour chaque état : repos (Idle), flexion du coude gauche (LE), saisie de la main gauche (LH), flexion du coude droit (RE), saisie de la main droite (RH).

L'invention permet un apprentissage en ligne, non supervisé, d'un modèle prédictif de commande, et peut être mise en œuvre pour tout système de type BCI, y compris les dispositifs dans lesquels le décodage est transmis aux nerfs de la colonne vertébrale ou aux muscles.

**Revendications**

**1.** Procédé d'apprentissage d'une interface neuronale directe, l'interface neuronale directe comportant des capteurs (21...211, 5) disposés autour du cerveau d'un utilisateur, et configurés pour détecter des signaux électrophysiologiques, représentatifs d'une activité neuronale de l'utilisateur, l'interface étant configurée pour :

- commander un actionneur (6), en mettant en œuvre un modèle prédictif de commande, le modèle prédictif de commande étant configuré pour générer un signal de commande de l'actionneur à partir de signaux électrophysiologiques détectés ;
- estimer un état de satisfaction de l'utilisateur en mettant en œuvre un modèle de décodage, le modèle de décodage étant configuré pour estimer l'état de satisfaction de l'utilisateur à partir de signaux électrophysiologiques détectés ;

le procédé comportant les étapes suivantes :

- a) choix d'une tâche (k) à effectuer, choisie parmi une liste de tâches prédéterminée $(1 ... k ...K)$ ;
- b) instruction, à l'utilisateur, d'imaginer une exécution de la tâche choisie lors de l'étape a) et suite à l'instruction, à l'aide de l'unité de traitement (3) :

  • acquisition des signaux électrophysiologiques moteurs, et formation d'un tenseur d'observation ($\overline{X_n}$) à partir de caractéristiques des signaux électrophysiologiques ;
  • application du modèle prédictif de commande (F) au tenseur d'observation, pour générer un signal de commande ($Y_n$), pour piloter l'actionneur, le signal de commande étant représentatif d'une tâche prédite ;

- c) réitération des étapes a) et b) durant plusieurs époques temporelles (n), à chaque époque étant assignée une tâche choisie lors de l'étape a) et une tâche prédite durant l'étape b) ;
- d) sélection d'époques auxquelles la tâche prédite lors de l'étape b) est identiques aux tâches prédites lors de l'étape b) d'un nombre minimal d'époques précédentes successives, les époques ainsi sélectionnées formant une séquence (v) ;
- e) pour chaque époque sélectionnée dans l'étape d), assignation d'un label ($y_{NR,n}$) représentant une comparaison de la tâche prédite lors de l'étape b), avec la tâche choisie lors de l'étape a), le label étant choisi parmi plusieurs valeurs possibles ;
- f) formation d'un vecteur de labels ($Y_{NR,v}$) à partir des labels assignés lors de l'étape e) ;
- g) formation d'un tenseur d'apprentissage ($\overline{X_v}$) à partir des tenseurs d'observation formés pour chaque époque sélectionnée dans l'étape d) ;
- h) mise à jour du modèle de décodage par régression entre le vecteur de labels ($Y_{NR,v}$) et le tenseur d'apprentissage ($\overline{X_v}$);

le procédé étant **caractérisé en ce que** l'étape h) comporte :

- définition d'un critère de pondération pour chaque époque sélectionnée (n) ;
- assignation d'un poids ($w_n$) à chaque époque sélectionnée (n), le poids étant défini en fonction du critère de pondération, en fonction duquel à deux époques différentes, de la séquence (v), pour lesquelles le critère de pondération est différent, sont assignés deux poids différents ;

et **en ce que** la formation du modèle de décodage est effectuée en fonction du poids respectivement assigné à chaque époque sélectionnée.

**2.** Procédé selon la revendication 1, dans lequel dans le poids assigné à une époque dépend de la tâche sélectionnée durant ladite époque.

**3.** Procédé selon la revendication 2, dans lequel l'étape h) comporte :

- hi) subdivision de chaque tâche (k), respectivement associée à chaque époque sélectionnée, en plusieurs sous-classes (*l*), correspondant respectivement à chaque valeur possible du label ($y_{NR,n}$) suite à l'étape e), de façon que la sous-classe attribuée à chaque époque corresponde à la valeur prise par le label pour la tâche (k) choisie lors de l'étape a) de ladite époque ;

- hii) assignation d'un poids ( $w_v^l$ ) à chaque sous-classe (*l*), le poids étant défini en fonction du critère de pondération;

- hiii) assignation du poids ($w_n$) à chaque époque (n), le poids de chaque époque correspondant au poids ( $w_v^l$ ) assigné, lors de la sous-étape hii), à la sous-classe attribuée à ladite époque lors de la sous-étape hi).

**4.** Procédé selon la revendication 3, dans lequel :

- les étapes a) à d) sont mises en œuvre durant plusieurs séquences successives ;
- les étapes e) à h) sont est mises en œuvre pour chaque séquence ;
- dans la sous-étape hii), le critère de pondération comporte une fréquence d'occurrence de chaque sous-classe, le poids de chaque époque est d'autant plus élevé que le nombre d'occurrences de la sous-classe, suite aux séquences successives effectuées, est faible.

**5.** Procédé selon la revendication 4 comportant, après chaque nouvelle séquence (v), une mise à jour d'un nombre total d'occurrences pondéré pour chaque sous-classe ( $N_v^l$ ), la mise à jour comportant, pour chaque sous-classe (*l*) :

- détermination d'un nombre d'occurrences ( $n_v^l$ ) dans la nouvelle séquence (v) ;

- pondération du nombre d'occurrences, au cours de la nouvelle séquence, par le poids ( $w_v^l$ ) respectivement assigné à sous-classe dans la nouvelle séquence ;
- sommation du nombre d'occurrences pondéré de la sous-classe, pour la nouvelle séquence, au nombre total pondéré pour chaque sous-classe ( $N_{v-1}^l$ ) résultant de la séquence précédente ( $N_{v-1}^l$ ), ce dernier étant multiplié par un facteur d'oubli (λ).

**6.** Procédé selon l'une quelconque des revendications précédentes, dans lequel le modèle de décodage est défini par régression multivariée, comportant un calcul d'un tenseur de covariance croisée entre le tenseur d'apprentissage ($\overline{X_v}$) et le vecteur de labels ($Y_{NR,v}$), le tenseur de covariance croisé ( $C_u^{XY_{NR}}$ ) de chaque séquence étant établi à partir d'un produit :

- du tenseur d'apprentissage ;
- du vecteur de labels;
- des poids ($w_n$) assignés à chaque époque.

**7.** Procédé selon la revendication 6, dans lequel

- les étapes a) à d) sont répétées de façon à former plusieurs séquences successives, à chaque séquence étant un rang chronologique (v) ;
- les étapes e) à h) sont est mises en œuvre pour chaque séquence ;
- lors de l'étape h), le modèle de décodage est établi à partir de deux séquences consécutives, à partir d'une somme du tenseur de covariance croisée établi pour la séquence de rang supérieur (v) et du tenseur de covariance croisée établi pour la séquence de rang inférieur (v-1) multiplié par un facteur d'oubli (λ).

**8.** Procédé selon l'une quelconque des revendications 6 ou 7, dans lequel :

- le tenseur d'apprentissage prend la forme d'une matrice, dont une dimension est le nombre d'époques (M') par séquence ;
- dans chaque étape h), relative à chaque séquence, les poids ($w_n$) forment une matrice diagonale ($diag(W_v)$), chaque terme de la matrice diagonale correspondant au poids ($w_n$) assigné à l'époque (n) respectivement exécutée lors de ladite séquence.

9. Procédé selon l'une quelconque des revendications précédentes, comportant également :

- j-i) formation d'un tenseur d'observation auxiliaire ($X_{NR,n}$) à partir des signaux électrophysiologiques détectés lors de chaque époque sélectionnée dans l'étape d) ;
- j-ii) application d'un modèle de décodage au tenseur d'observation auxiliaire de façon à estimer un label ($\hat{y}_{NR,n}$) pour chaque époque ;
- j-iii) pondération de la valeur du label estimé pour chaque époque, par le poids ($w_n$) assigné à chaque époque lors de l'étape h) ;
- j-iv) formation d'un histogramme des valeurs pondérées de chaque label estimé ;
- j-v) calcul d'un fractile inférieur et d'un fractile supérieur à partir de l'histogramme ;
- j-vi) association d'un seuil inférieur ($q_{inf}$) et d'un seuil supérieur ($q_{sup}$) respectivement à partir du fractile inférieur et du fractile supérieur, de façon qu'un label estimé dont la valeur est inférieure ou supérieure respectivement au seuil inférieur ou au seuil supérieur est considéré comme un label ayant une valeur d'erreur ou une valeur correcte ;

les étapes j-i) à j-vi) étant mise en œuvre par l'unité de traitement (3).

10. Procédé d'apprentissage d'une interface neuronale directe, l'interface neuronale directe comportant des capteurs ($2_1$ ... $2_{I1}$, 5) disposés autour du cerveau d'un utilisateur, et configurés pour détecter des signaux électrophysiologiques, représentatifs d'une activité neuronale de l'utilisateur, l'interface étant configurée pour :

- commander un actionneur (6), en mettant en œuvre un modèle prédictif de commande, le modèle prédictif de commande étant configuré pour générer un signal de commande de l'actionneur à partir de signaux électrophysiologiques détectés ;
- estimer un état de satisfaction de l'utilisateur en mettant en œuvre un modèle de décodage, le modèle de décodage étant configuré pour estimer l'état de satisfaction de l'utilisateur à partir de signaux électrophysiologiques détectés, le modèle de décodage étant établi à partir d'un procédé selon l'une quelconque des revendications 1 à 10 ;
le procédé comportant :

- i) choix, par l'utilisateur, d'une tache mentale (k) à effectuer, choisie parmi une liste de tâches prédéterminée (1 ... k ... K);
- ii) exécution, par l'utilisateur, de la tâche choisie lors de l'étape i) et, au cours de l'exécution, acquisition des signaux électrophysiologiques, issus des différents capteurs ;
- iii) durant l'exécution de la tâche choisie, mise en œuvre du modèle prédictif de commande, de façon à générer un signal de commande ($Y_n$) ;
- v) suite à la génération du signal de commande, estimation d'un label ($\hat{y}_{NR,n}$), correspondant à un état de satisfaction de l'utilisateur ;
- vi) réitération des étapes i) à v) durant un nombre prédéterminé d'époques temporelles (n), lesdites époques formant une séquence (u) ;
- vii) sélection d'époques en fonction du niveau de satisfaction de l'utilisateur estimé lors de chaque étape v) ;
- viii) formation d'un tenseur d'apprentissage ($\overline{X_u}$) à partir des signaux électrophysiologiques détectés au cours des époques sélectionnées dans l'étape vii) et d'un tenseur de commande ($Y_u$) à partir des signaux de commande générés au cours des époques sélectionnées dans l'étape vii) ;
- ix) mise à jour du modèle prédictif de commande en fonction du tenseur d'apprentissage et du tenseur de commande résultant de vii), pour la séquence (u);

les étapes iii) à ix) étant mises en œuvre par l'unité de traitement à partir de signaux électrophysiologiques détectés ;
le procédé étant **caractérisé en ce que** l'étape ix) comporte :

- définition d'un critère de pondération pour chaque époque sélectionnée dans l'étape vii) ;

- assignation d'un poids ($w_n$) à chaque époque, le poids étant défini en fonction du critère de pondération pour ladite époque, en fonction duquel à deux époques différentes, pour lesquelles le critère de pondération est différent, sont assignés deux poids différents ;

le procédé étant tel que la formation du modèle prédictif de commande est effectuée en fonction du poids respectivement assigné à chaque époque.

11. Procédé selon la revendication 10, dans lequel :

- les étapes i) à ix) sont mises en œuvre durant plusieurs séquences successives ;
- le critère de pondération comporte une fréquence d'occurrence de chaque tâche, le poids de chaque époque est d'autant plus élevé que le nombre d'occurrences de la tâche, suite aux séquences successives effectuées, est faible.

12. Procédé selon l'une quelconque des revendications 10 ou 11, dans lequel le critère de pondération est une performance d'apprentissage, le procédé comportant :

- détermination d'un indicateur de performance d'apprentissage de chaque tâche suite à chaque époque ;
- détermination du poids de chaque tâche en fonction du critère de performance d'apprentissage de la tâche.

13. Procédé selon l'une quelconque des revendications 10 à 12, dans lequel le critère de pondération est une qualité des signaux collectés à chaque séquence, le procédé comportant :

- détermination d'un critère de qualité des signaux collectés à chaque séquence ;
- détermination du poids de chaque tâche en fonction du critère de qualité des signaux.

14. Interface neuronale directe, l'interface neuronale directe comportant des capteurs ($2_1...2_{11}$, 5) configurés pour être disposés autour du cerveau d'un utilisateur, et configurés pour détecter des signaux électrophysiologiques, représentatifs d'une activité neuronale de l'utilisateur, l'interface étant configurée pour :

- commander un actionneur (6), en mettant en œuvre un modèle prédictif de commande, le modèle prédictif étant configuré pour générer un signal de commande de l'actionneur à partir de signaux électrophysiologiques détectés ;
- estimer un état de satisfaction de l'utilisateur en mettant en œuvre un modèle de décodage, le modèle de décodage étant configuré pour estimer l'état de satisfaction de l'utilisateur à partir de signaux électrophysiologiques détectés ;

l'interface comportant une unité de traitement (3), configurée pour acquérir des signaux électrophysiologiques lors de chaque étape b) d'un procédé selon l'une quelconque des revendications 1 à 9, et pour mettre en œuvre les étapes c) à h) dudit procédé.

15. Interface neuronale directe, l'interface neuronale directe comportant des capteurs ($2_1...2_{11}$, 5) configurés pour être disposés autour du cerveau d'un utilisateur, et configurés pour détecter des signaux électrophysiologiques, représentatifs d'une activité neuronale de l'utilisateur, l'interface étant configurée pour :

- commander un actionneur (6), en mettant en œuvre un modèle prédictif, le modèle prédictif étant configuré pour générer un signal de commande de l'actionneur à partir de signaux électrophysiologiques détectés ;
- estimer un état de satisfaction de l'utilisateur en mettant en œuvre un modèle de décodage, le modèle de décodage étant configuré pour estimer l'état de satisfaction de l'utilisateur à partir de signaux électrophysiologiques détectés ;

l'interface comportant une unité de traitement, configurée pour acquérir des signaux électrophysiologiques lors de chaque étape ii) d'un procédé selon l'une quelconque des revendications 10 à 13, et pour mettre en œuvre les étapes iii) à ix) dudit procédé.

**Fig. 1**

**Fig. 2A**

**Fig. 2B**

**Fig. 2C**

$$q_{inf} = fractile_{inf}(\hat{y}_{NR})$$

$$q_{sup} = fractile_{sup}(\hat{y}_{NR})$$

$\hat{y}_{NR}$

**Fig. 3**

**Fig. 4A**

**Fig. 4B**

(s)

**Fig. 5A**

**Fig. 5B**

**Fig. 5C**

**Fig. 5D**

**Fig. 5E**

**Fig. 5F**

**Fig. 6A**

**Fig. 6B**

**Fig. 6C**

**Fig. 6D**

**Fig. 6E**

**Fig. 6F**

Europäisches Patentamt

European Patent Office

Office européen des brevets

## RAPPORT DE RECHERCHE EUROPEENNE

Numéro de la demande

EP 25 22 7311

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A,D | EP 4 024 170 A1 (COMMISSARIAT ENERGIE ATOMIQUE [FR]) 6 juillet 2022 (2022-07-06) * alinéas [0011] - [0014], [0017], [0020], [0042] - [0045], [0052] - [0055], [0058] - [0060]; figures 1A, 1B, 2, 3 * ----- | 1-15 | INV. G06F3/01 A61B5/00 A61B5/372 |
| A,D | EP 3 985 530 A1 (COMMISSARIAT ENERGIE ATOMIQUE [FR]) 20 avril 2022 (2022-04-20) * le document en entier * ----- | 1-15 | |
| A,D | EP 3 563 218 B1 (COMMISSARIAT ENERGIE ATOMIQUE [FR]) 6 janvier 2021 (2021-01-06) * le document en entier * ----- | 1-15 | |

**DOMAINES TECHNIQUES RECHERCHES (IPC)**

G06F
A61B
G06N

Le présent rapport a été établi pour toutes les revendications

| Lieu de la recherche | Date d'achèvement de la recherche | Examinateur |
|---|---|---|
| Munich | 17 avril 2026 | Fournier, Nicolas |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**   EP 25 22 7311

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

17-04-2026

| Document brevet cité au rapport de recherche | | Date de publication | Membre(s) de la famille de brevet(s) | | Date de publication |
|---|---|---|---|---|---|
| EP  4024170 | A1 | 06-07-2022 | EP | 4024170 A1 | 06-07-2022 |
| | | | FR | 3118413 A1 | 01-07-2022 |
| | | | US | 2022207424 A1 | 30-06-2022 |
| EP  3985530 | A1 | 20-04-2022 | EP | 3985530 A1 | 20-04-2022 |
| | | | FR | 3115124 A1 | 15-04-2022 |
| | | | US | 2022110570 A1 | 14-04-2022 |
| EP  3563218 | B1 | 06-01-2021 | EP | 3563218 A1 | 06-11-2019 |
| | | | FR | 3061318 A1 | 29-06-2018 |
| | | | US | 2019320924 A1 | 24-10-2019 |
| | | | WO | 2018122518 A1 | 05-07-2018 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 9480583 B
- EP 3985530 A
- EP 4024170 A
- EP 3789852 A
- FR 2415310
- EP 3563218 A

**Littérature non-brevet citée dans la description**

- **ELISEYEV A** ; **AKSENOVA T**. Recursive N-way Partial Least Squares for Brain Computer Interface. *PIOS ONE*, July 2013, vol. 8 (7)
- Brain-Computer Interface with cortical electrical activity recording. **YELISYEYEV A**. Human health and pathology. Université de Grenoble, 2011
- **ROUANNE**. Unsupervised adaptation of an ECoG based brain-computer interface using neural correlates of task performance. *Nature scientific reports*, 2012, vol. 12, 21316
- **MESTAIS C. et al.** WIMAGINE : Wireless 64-Channel ECoG recording implant for long term clinical applications. *IEEE Transactions on neural systems and rehabilitation engineering*, January 2015, vol. 23 (1)